(19) Europäisches Patentamt
European Patent Office
Office européen des brevets

(11) EP 2 334 257 B1

(12) EUROPEAN PATENT SPECIFICATION

(45) Date of publication and mention
of the grant of the patent:
31.05.2017 Bulletin 2017/22

(51) Int Cl.:
A61F 2/02 (2006.01)     A61L 27/36 (2006.01)

(21) Application number: 09815305.9

(22) Date of filing: 18.09.2009

(86) International application number:
PCT/US2009/057568

(87) International publication number:
WO 2010/033860 (25.03.2010 Gazette 2010/12)

(54) **BIONANOCOMPOSITE FOR TISSUE REGENERATION AND SOFT TISSUE REPAIR**

BIONANOKOMPOSIT ZUR GEWEBEREGENERIERUNG UND WEICHGEWEBEREPARATUR

BIONANOCOMPOSITE DE RÉGÉNÉRATION DE TISSU ET DE RÉPARATION DE TISSU MOU

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 18.09.2008 US 192418 P

(43) Date of publication of application:
22.06.2011 Bulletin 2011/25

(73) Proprietor: The Curators Of The University Of
Missouri
Columbia, Missouri 65211-2015 (US)

(72) Inventors:
• GRANT, Sheila Ann
Columbia, Missouri 65203 (US)
• DEEKEN, Corey Renee
Columbia, Missouri 65202 (US)
• RAMSHAW, Bruce John
Columbia, Missouri 65203 (US)
• BACHMAN, Sharon Liebe
Columbia, Missouri 65201 (US)

• RAMASWAMY, Archana
Columbia, Missouri 65201 (US)
• FEARING, Nicole Marie
Columbia, Missouri 65211 (US)

(74) Representative: Smaggasgale, Gillian Helen
WP Thompson
138 Fetter Lane
London EC4A 1BT (GB)

(56) References cited:
EP-A1- 1 698 358       US-A1- 2001 000 804
US-A1- 2006 204 441    US-A1- 2006 257 377
US-A1- 2007 128 420    US-A1- 2007 207 182
US-A1- 2007 244 568    US-B1- 6 646 103

• HAIDEKKER MARK A ET AL: "Influence of gold
nanoparticles on collagen fibril morphology
quantified using transmission electron
microscopy and image analysis", BMC MEDICAL
IMAGING, BIOMED CENTRAL, LONDON, GB, vol.
6, no. 1, 31 May 2006 (2006-05-31), page 4,
XP021015772, ISSN: 1471-2342, DOI:
10.1186/1471-2342-6-4

**Description**

**FIELD OF INVENTION**

The present invention relates to prosthetic materials, methods of fabrication, and applications thereof. More specifically, the present invention relates to a series of biocompatible materials that can be used in soft tissue repair in a living body.

**BACKGROUND OF INVENTION**

The availability of biocompatible materials for soft tissue repair applications such as hernia repair, meniscus tissue replacement, and vascular grafts, is a critical issue for the medical society due to the large number of patients requiring these types of repairs. For example, millions of inguinal hernia repairs are performed each year worldwide with 750,000 inguinal and 150,000 ventral repairs performed in the United States alone.

Hernias are by definition a breakdown of the tough connective tissue that encases the abdominal musculature, known as fascia. As a result, there is a bulging of the intra-abdominal viscera through the abdominal wall defect, with a wide variation of resulting symptoms. This bulge may be asymptomatic, unsightly, and may cause pain when contracting the abdominal musculature. Some patients have chronic unremitting pain. The most concerning scenario is entrapment of the viscera within the defect, known as incarceration, which can be quite painful, cause bowel obstruction, or lead to strangulation of the bowel and potential intestinal death, resulting in a surgical emergency.

To help decrease the rate of hernia recurrence, a prosthetic mesh material is utilized to repair the hernia defect. The role of mesh in these repairs is to provide a tension-free bridge between the fascial defects and/or reinforcement of the fascia. The first mesh used was made of nylon, which was soon supplanted by other synthetic materials, such as polyester, polypropylene and polyethylene. The original thought was that a heavy mesh was preferable to prevent rupture and re-herniation. The fact that the polypropylene induced a fibrotic, inflammatory response was considered beneficial. The theory was that more scarring would lead to a stronger abdominal wall and less recurrence. Despite stimulating an intense cellular reaction, the mesh was considered to be biologically inert and stable in vivo.

In the past decade, this theory has been challenged. It is becoming recognized that mesh shrinkage, especially of heavy-weight mesh, can result in up to a 66% reduction in the surface area. Mesh shrinkage may uncover the original defect and lead to a recurrence of the hernia. When mesh is placed in the abdominal wall, the robust fibrotic response may cause chronic pain associated with either nerve entrapment in the scar plate or mesh contraction. Abdominal wall mobility may become limited. The specter of infection looms large, as these prosthetics frequently cannot be cleared of bacteria by the phagocytes, and mesh removal may be required.

Methods of producing artificial composite tissue constructs that permit coordinated motion are discussed in US2006257377. Biocompatable structural matrices having sufficient rigidity to provide structural support for cartilage-forming cells and bone-forming cells are used. Biocompatable flexible matrices seeded with muscle cells are joined to the structural matrices to produce artificial composite tissue constructs that are capable of coordinated motion.

In general, implanted biomaterials utilized for soft tissue repair suffer from poor tissue integration, which permits sliding and rubbing of the material on the cells and tissues. This lack of control at the biomaterial-tissue interface and the body's natural response to a foreign body results in repeated cellular injury and a chronic inflammatory response. This may lead to decreased function, chronic pain, and eventual implant removal. New soft tissue repair materials have utilized collagen scaffolds, but purified collagen is mechanically weak and chemically crosslinked collagen has inadequate biocompatibility.

Therefore, there is a need to provide a new and improved implant material that combats the problems of mesh shrinkage, infection, and recurrences, while promoting tissue integration and improving the overall biocompatibility when used in soft tissue repair. There is another need to provide a new and improved scaffold material for tissue re-engineering.

**SUMMARY OF INVENTION**

According to the present invention there is provided a bionanocomposite comprising: decellularized tissue; and nanomaterial crosslinked with the decellularized tissue and functionalized with surface functional groups capable of bonding with tissue; the nanomaterial is gold nanoparticles, gold nanorods, gold nanofibers, silver nanoparticles, silver nanorods, silver nanofibers, platinum nanoparticles, platinum nanorods, platinum nanofibers, titania nanoparticles, titania nanorods, titania nanofibers, silica nanoparticles, silica nanorods, silica nanofibers, alumina nanoparticles, alumina nanorods, alumina nanofibers, calcium phosphate nanoparticles, calcium phosphate nanorods, calcium phosphate nanofibers, BaTiO3 nanoparticles, a BaTiO3 nanorods, BaTiO3 nanofibers, polycaprolactone nanofibers, polyglycolic acid nanofibers, polylactic acid nanofibers, polylacticglycolic acid nanofibers, polydoxanone nanofibers, trimethylene carbonate nanofibers, or combinations thereof; wherein the gold nanoparticles are functionalized with -COOH groups, - OH groups, methionine, mercaptomethylamine, mercaptoethylamine (MEA), mercaptopropylamine, mercaptobutylamine, or a combination thereof, , the silicon carbide nanowires are functionalized with COOH groups, OH groups, aminopropyl-triethoxysilane, plasma polymerization with allyl amine, plasma polymerization with acrylic acid, plasma polymerization with hydroxyethyl methacrylate, and the polycaprolactone nanofibers are functionalized other than by aminolysis.

The bionanocomposite is useful in hernia repair, meniscus tissue replacement, or vascular grafts.

Also disclosed is an implant for treating a soft tissue injury comprising the bionanocomposite of the above first aspect.

**BRIEF DESCRIPTION OF DRAWINGS**

Figures 1A and 1B are the Scanning Electron Micrographs ("SEM") of natural tissue before decellularization and the decellularized tissue respectively.

Figures 2A and 2B are the SEMs of decellularized tissue crosslinked with silicon carbide nanowires ("SiCNW"), where Figure 2A shows SiCNW on the surface of the decellularized tissue and Figure 2B shows SiCNW in close-up.

Figures 3A and 3B are the SEMs of decellularized tissue crosslinked with gold nanoparticles ("AuNP"), where Figure 3A shows AuNP on the surface of the decellularized tissue and Figure 3B shows AuNP without surrounding decellularized tissue.

Figure 4 depicts the results of the flow cytometry experiments that demonstrate the biocompatibility of the nanomaterials.

Figure 5 shows cells proliferating within the decellularized porcine tendon tissue, SiC-nanowire crosslinked tissue, and Au-nanoparticle crosslinked tissue at Day 3, 7, 14 of the bioreactor study.

Figure 6 depicts the DNA content inside the decellularized porcine tendon tissue, SiC-nanowire crosslinked tissue, and Au-nanoparticle crosslinked tissue as a measure of cellularity after 3, 7, and 14 days in the bioreactor.

Figure 7 depicts the GAG content (normalized for DNA) inside the decellularized porcine tendon tissue, SiC-nanowire crosslinked tissue, and Au-nanoparticle crosslinked tissue after 3, 7, and 14 days in the bioreactor.

Figure 8A is a graph of the tensile strength at yield (MPa) of the natural tissue, EDC crosslinked tissue, Au-nanoparticle crosslinked tissue, and SiC-nanowire crosslinked tissue.

Figure 8B is a graph of the μg hydroxyproline/mg tissue released upon digestion with collagenase for natural tissue, EDC crosslinked tissue, Au-nanoparticle crosslinked tissue, and SiC-nanowire crosslinked tissue.

Figure 9 is a graph of the Young's modulus (MPa) for untreated tissue, decellularized tissue, EDC crosslinked tissue, EDC-double crosslinked tissue, Au-nanoparticle crosslinked tissue, Surgisis, and Permacol.

Figure 10A is a photograph of a H&E stain of a representative scaffold after implantation into a tissue.

Figure 10B is a photograph of a H&E stain (20x) of a Permacol scaffold explant after one month in vivo.

Figure 10C is a photograph of a H&E stain (20x) of a Surgisis scaffold explant after one month in vivo.

Figure 10D is a photograph of a H&E stain (20x) of an AuNP-crosslinked scaffold explant after one month in vivo.

Figure 10E is a photograph of a H&E stain (20x) of an EDC-crosslinked scaffold explant after one month in vivo.

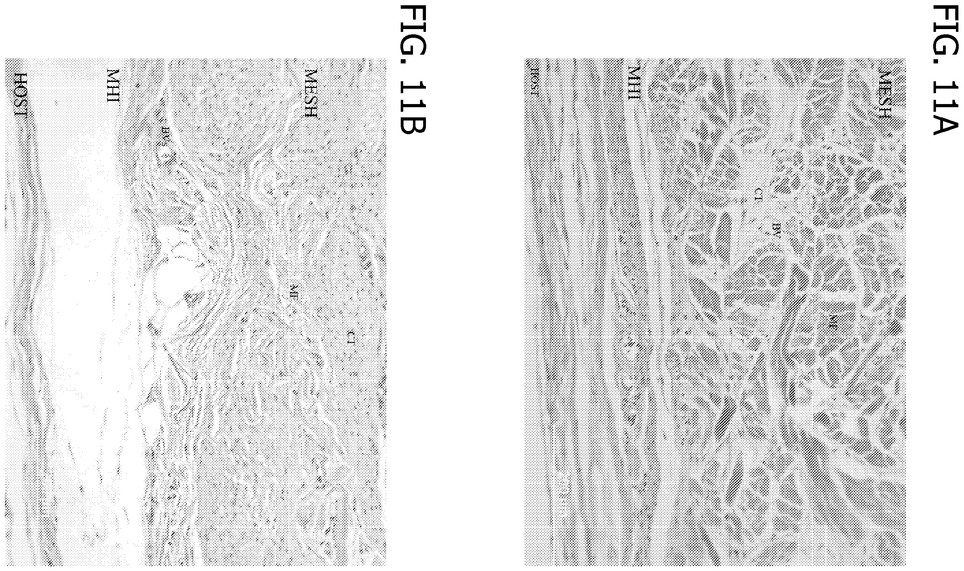

Figure 11A is a photograph of a H&E stain (20x) of a Permacol scaffold explant after six months in vivo.

Figure 11B is a photograph of a H&E stain (20x) of a Surgisis scaffold explant after six months in vivo.

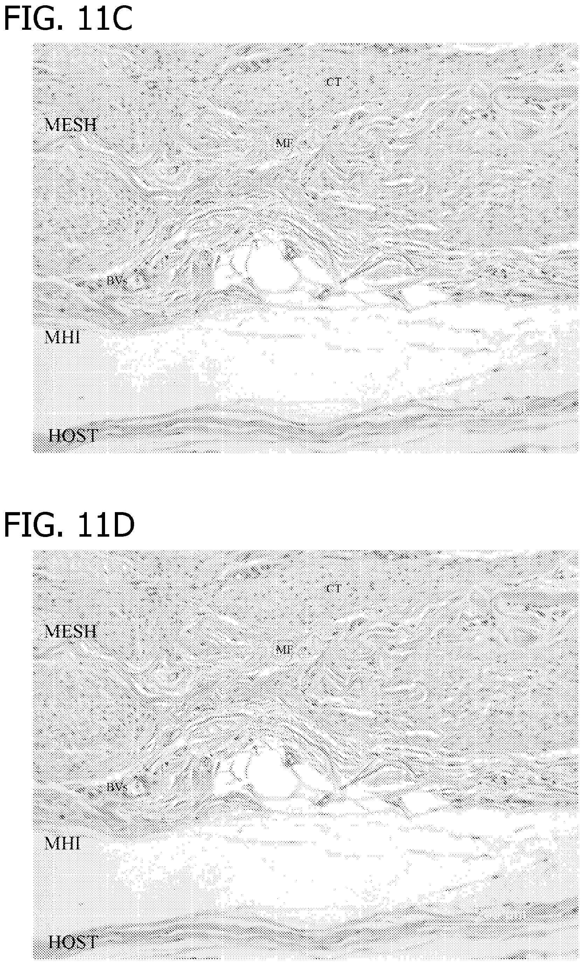

Figure 11C is a photograph of a H&E stain (20x) of a diaphragm scaffold explant after six months in vivo.

Figure 11D is a photograph of a H&E stain (20x) of a AuNP-diaphragm scaffold explant after six months in vivo.

Figure 12 is a schematic diagram illustrating projection of a bionanocomposite onto a planar surface.

**DETAILED DESCRIPTION OF INVENTION**

Unless otherwise defined, all technical and scientific terms used herein have the same meaning as commonly understood by one of ordinary skill in the art to which this invention belongs.

The inventive Bionanocomposite involves crosslinking nanomaterials to decellularized tissues, which improves the overall strength of the material while promoting tissue in-growth when utilized for soft tissue repair. The invention selects decellularized tissues as the biologic scaffolds over pure collagen or other manufactured polymer mesh materials currently available on the market. Among many advantages, the decellularized tissue includes a mixture of collagen, elastin, and other structural and functional proteins that constitute the extracellular matrix. The extracellular matrix ("ECM") is an ideal scaffold material because it naturally possesses the bioactive components and structure necessary to support cell adhesion and tissue ingrowth, initiate angiogenesis, and promote constructive tissue regeneration. As ECM scaffolds degrade, growth factors and peptides are released. These elements possess antimicrobial properties that ward off potential pathogens, and they also influence angiogenesis and tissue remodeling through the recruitment of endothelial and bone marrow-derived cells.

Decellularized Tissue

The decellularized tissue may be obtained from treatment of biological tissue, which may be harvested from either allograft or xenograft. The tissue is decellularized in that cells and cellular remnants are removed while the extracellular matrix components remains intact. A variety of biological tissue donor sources may be employed, such as human (dermis, tensor fascia lata, blood vessels, and amniotic membrane), porcine (small intestine submucosa, dermis, blood vessels, and bladder), bovine (dermis, blood vessels, and pericardium), and equine (blood vessels and pericardium), which have been studied for other purposes. Many of these materials provide desirable degradation characteristics and when implanted either alone or once crosslinked to nanoparticles, can release growth factors and peptides that posses antimicrobial properties, enhance angiogenesis, and aid tissue remodeling by attracting endothelial and bone marrow-derived cells to the implant site.

In many instances, the tissue may be selected according to its handling properties for surgical manipulation and mechanical properties (strength, elasticity, size, etc.) required for the targeted soft tissue repair application. For example, the thickness of the tissue affects its handling properties and tissues having a thickness of from about 0.5 mm to about 3 mm; from about 0.5 mm to about 2 mm; from about 0.5 mm to about 1.5 mm; or from about 0.8 mm to about 1.2 mm. are preferred. Also, the tensile strength of the decellularized tissue measured at yield ranges from about 16 MPa to about 25 MPa; from about 16.5 MPa to about 25 MPa; from about 17 MPa to about 25 MPa; from about 17 MPa to about 22 MPa; from about 17.5 MPa to about 25 MPa; from about 18 MPa to about 25 MPa; or from about 18.5 MPa to about 25 MPa. For commercialization purposes, a user may also consider whether large quantities of the tissue can be easily obtained and processed.

The mechanical and chemical properties of the decellularized material desirably do not change significantly once implanted in an animal. For example, the viscoelasticity of the decellularized material does not change significantly as cells from the surrounding tissue infiltrate the decellularized material and it degrades. In order to have a composite that has a desired viscoelasticity, the tissue should have an appropriate degradation rate. Further, the viscoelasticity can be measured by the Young's modulus wherein a higher value means the tissue is stiffer and a lower value means the tissue is less stiff. Preferably, the viscoelasticity of the bionanocomposite is from about 100 MPa to about 200 MPa; from about 125 MPa to about 200 MPa; from about 150 MPa to about 200 MPa; or from about 160 MPa to about 200 MPa.

In addition to these considerations, the degradation rate of the tissue can also influence the selection of a particular tissue. When utilized for soft tissue repair, it is important that the selected natural tissue is degraded by the body at a rate that matches the healing rate of the defective area so that it can serve as an effective repair material without inciting a chronic inflammatory response.

The selected biological tissues needs to be processed to remove native cells, i.e. "decellularized" in order to prevent an immune response when it is utilized as a soft tissue repair material. (Gilbert et al. Decellularization of tissues and organs. Biomaterials 2006;27:3675-3683) The decellularization process may be optimized for each species and type of tissue. Successful decellularization is characterized by the removal of cellular nuclei and remnants with the retention of natural extracellular matrix components (collagen, elastin, growth factors, etc.) and overall tissue structure (collagen architecture). (Gilbert et al.) For example, from about 80% to 100%, from about 85% to about 100%, from about 90% to about 100%, or from about 95% to about 100% of the cellular nuclei and remnants are removed from the tissue. Further, the decellularized material can contain from about 0.1 % to about 20%; from about 0.1 % to about 15%; from about 0.1 % to about 10%; from about 0.1 % to about 5% of the original cellular material after decellularization. The collagen structure is ideal for cell attachment and infiltration. Thus, maintaining the collagen structure is desirable during the decellularization process. For example, the collagen structure has pore size from about 1 nm to about 100 nm. Further, the collagen structure has a porosity of from about 10% to about 90%; from about 20% to 90%; from about 30% to about 90%; from about 30% to about 80%; or from about 40% to about 80%.

The decellularizing process can take the form of physical (sonication, freezing, agitation, etc.), chemical (acids, ionic, non-ionic, and zwitterionic detergents, organic solvents, etc.), and enzymatic (protease, nuclease, etc.) treatments or a combination thereof and may employ any procedure commonly practiced in the field. (Gilbert et al.) Physical methods for decellularization include freezing, direct pressure, sonication, and agitation; these methods need to be modified depending on the particular tissue. Chemical methods include treatment with an acid, a base, a non-ionic detergent, an ionic detergent, a zwitterionic detergent, an organic solvent, a hypotonic solution, a hypertonic solution, a chelating agent, or a combination thereof.

The acid or base solubilizes cytoplasmic components of cell and disrupts nucleic acids. Exemplary acids and bases are acetic acid, peracetic acid, hydrochloric acid, sulfuric acid, ammonium hydroxide or a combination thereof. Treatment with non-ionic detergents disrupts lipid-lipid and lipid-protein interactions, while leaving protein-protein interactions intact. An exemplary non-ionic detergent is Triton X-100. An ionic detergent solubilizes cytoplasmic and nuclear cellular membranes and tends to denature proteins. Exemplary ionic detergents are sodium dodecyl sulfate, sodium deoxycholate, Triton X-200, or a combination thereof. A zwitterionic detergent treatment exhibits properties of on-ionic and ionic detergents. Exemplary zwitterionic detergents are 3-[3-cholamidopropyl)dimethylammonio]-1-propanesulfonate (CHAPS), sulfobetaine-10 (SB-10), sulfobetaine-16 (SB-16), or a combination thereof. Tri(n-butyl)phosphate is an organic solvent that disrupts protein-protein interactions. Chelating agents bind divalent metallic ions that disrupt cell adhesion to the extracellular matrix. Exemplary chelating agents are ethylenediamine tetraacetic acid (EDTA), ethylene glycol tetraacetic acid (EGTA), or a combination thereof.

The decellularization can also be carried out using enzymatic methods. Exemplary enzymes are trypsin, endonucleases, exonucleases, or a combination thereof. Trypsin cleaves peptide bonds on the C-side of arginine and lysine. Endonucleases catalyze the hydrolysis of the interior bonds of ribonucelotide and deoxyribonucleotide chains. Exonucleases catalyze the hydrolysis of the terminal bonds of ribonucleotide and deoxyribonucleotide chains. In various embodiments, the decellularization is performed by treatment with acetic acid, peracetic acid, hydrochloric acid, sulfuric acid, ammonium hydroxide, Triton X-100, sodium dodecyl sulfate, sodium deoxycholate, Triton X-200, 3-[3-cholamidopropyl)dimethylammonio]-1-propanesulfonate (CHAPS), sulfobetaine-10 (SB-10), sulfobetaine-16 (SB-16), tri(n-butyl)phosphate, EDTA, EGTA, or a combination thereof.

Generally, the decellularization process includes immersion of the desired tissue in an agent that can make the tissue acellular (i.e., the tissue contains no cells). The agent that makes the tissue acellular can be an acid, a solvent, a surface active agent, and the like. The concentration of the agent is from about 0.5% (v/v) to about 5% (v/v). In various preferred processes, the concentration of the agent is from about 1% (v/v) to about 2% (v/v). In some of the various embodiments, the tissue is immersed in the agent for about 6 hours to about 36 hours; from about 12 hours to about 30 hours; from about 18 hours to about 30 hours; or from about 20 hours to 28 hours. The decellularization process was performed at room temperature. In particular embodiments, the decellularization process can include immersion for 24 hours with agitation in the following solutions: (1) 0.1% (v/v) peracetic acid with 4% ethanol, (2) 1% (v/v) TritonX-100, (3) 1% (v/v) Triton X-100 with 1% (v/v) tributyl phosphate (TnBP), (4) 2% (v/v) TnBP, (5) 1% (v/v) TnBP, (6) 1% (w/v) sodium dodecyl sulfate (SDS), (7) 0.5% (w/v) SDS.

A combination of both physical and chemical treatments may be employed. This arrangement includes two substeps, decellularization and subsequent rinses. In the decellularization step, the selected biological tissue is submersed in a buffered solution containing an organic solvent, tri(n-butyl)phosphate (TnBP), with agitation, such as in an orbital shaker, for about 24 hours. The resulting tissue is then rinsed to remove residual solvent and cellular remnants. The rinsing solvents may be deionized water and about 70% ethanol consecutively for a period of time, such as about 24 hours each. The tissue:solution volume ratio is from about 1:500 to about 5:100; from about 1:200 to about 2:100; or about 1:100 throughout the decellularization and subsequent rinses.

Several tests may be employed to verify the effectiveness of the decellularization process, i.e., removal of all cells and cellular remnants such as DNA while leaving extracellular matrix ('ECM') components (such as collagen, elastin, fibronectin, laminin, and glycosaminoglycans) intact. For example, a standard histological staining with hematoxylin and eosin (H&E) may be performed to identify any cell nuclei remaining in the resulting tissue. For example, the decellularized material desirably will be substantially free of cell nuclei and cellular remnants. Preferably, when a representative section of the decellularized material (1 cm x 1 cm) is stained with H&E, the decellularized material will have less than about 20 cell nuclei remaining and be substantially free of cellular remnants wherein substantially free of cell nuclei and cellular remnants means less than 15; less than 12; less than 10; less than 8; or less than 5 nuclei or cell remnants in the field of view of the decellularized tissue. Further, the collagen structure of the decellularized material is substantially the same as the structure of the tissue before decellularization. Finally, the decellularized tissue is biocompatible. The biocompatibility of the tissue can be measured using flow cytometry wherein cells incubated with the decellularized tissue did not show a significantly higher cell death rate as compared to the same cells under the same conditions but without contacting a tissue. A significantly higher cell death rate occurs when statistical significance ($p < 0.05$) is measured. Microscopic analyses may be performed to verify that all fibroblasts and endothelial cells are successfully removed from the resulting tissue. Methyl green pyronin stain, which stains for DNA and RNA, may also be utilized to verify that remnants of DNA and RNA are effectively removed from the tissue during the extensive rinse sequence. Further histological analyses, such as Masson's Trichrome, Verhoeff-van Gieson, and Alcian Blue staining, may also be performed to verify that ECM components remain within the decellularized tissue.

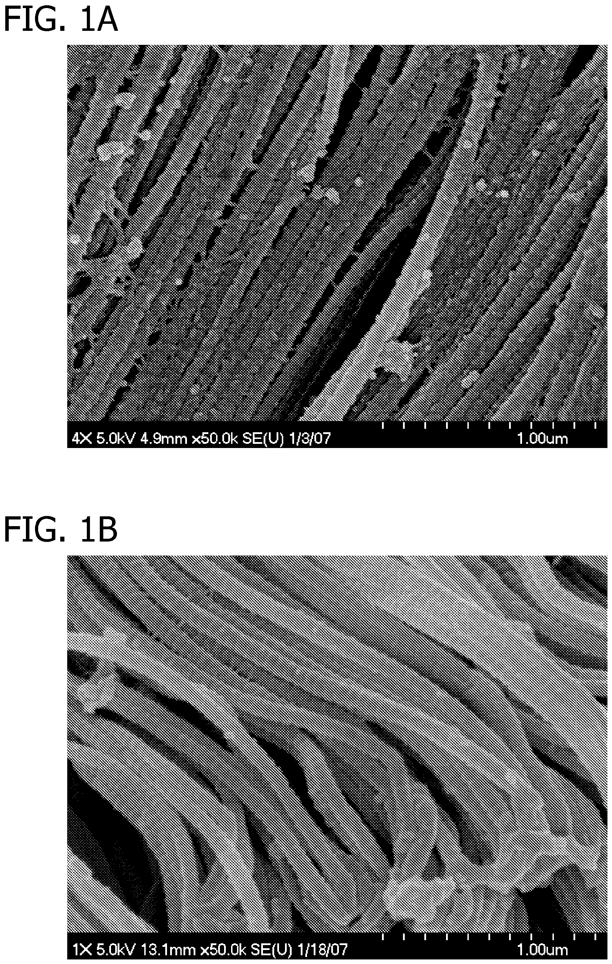

Further, scanning electron micrographs (SEMs) and collagenase assay results show that decellularization with 1% (v/v) TnBP did not significantly degrade the structure of the collagen in the porcine diaphragm tendon. SEMs were obtained of the porcine diaphragm tendon tissue before and after decellularization. Figure 1A is the Scanning Electron Micrograph ("SEM") of an exemplary biological (or nature) tissue, i.e., central tendon tissue of a porcine diaphragm. Figure 1B shows the SEM of this tissue after it was decellularized. These SEMs show that the decellularized material retains its fibrous structure as evidenced in Figure 1B.

Nanomaterials

Nanomaterials are incorporated to form the Bionanocomposite materials which improves the strength of the decellularized tissue and its resistance to degradation by the body, as well as to influence cellular behavior and biocompatibility. Prior studies have demonstrated that nanomaterials are more hydrophilic and possess an increased number of atoms and crystal grains at their surface compared to conventional materials. The large number of grains at the surface leads to increased surface roughness, surface area, and surface energy which are thought to contribute to an increase in protein adsorption and unfolding. For example, nanoscale ceramics, metals, and polymers have all been shown to improve cellular function compared to conventional materials. Webster TJ et al. J Biomed Mater Res 2000;51:475-483; Price RL, et al. Journal of Biomedical Materials Research Part A 2003;67A: 1284-1293; Webster TJ, et al. Biomaterials 2004;25:4731-4739; Park GE, et al. Biomaterials 2005;26:3075-3082; Thapa A, et al. Journal of Biomedical Materials Research Part A 2003;67A:1374-1383; Christenson EM, et al. Journal of Orthopaedic Research 2007;25:11-22.) These properties make nanomaterials ideally suited to enhance the biocompatibility and cell/tissue interaction with extracellular matrix-derived scaffolds.

The surface energy increase caused by the addition of nanoparticles is measured as compared to an otherwise identical biocomposite having micron-sized structures. Also, this surface energy increase is evidenced by increased protein adsorption as compared to an otherwise identical biocomposite having micron-sized structures. The identical biocomposite having micron-sized structures has the same matrix and chemical identity of the particles crosslinked to the matrix, but instead of nano-sized particles, rods, fibers, or wires, the composite has micron-sized particles, rods, fibers, or wires. The micron-sized material has a diameter or all dimensions of at least 100 nm. The protein adsorption can be measured by hematoxylin and eosin (H&E) stain of the composite followed by histology reading to quantify the amount of proteins adsorbed to the composition.

The nanomaterials employed in the invention may be selected from a variety of nanomaterials that are nontoxic and biocompatible such as gold, silver, silicon carbide, degradable polymers (polylactic acid/polyglycolic acid, polycaprolactone), carbon nanotubes, silicon, silica and combinations of coated nanomaterials. In some embodiments, the nanomaterial is gold nanoparticles, gold nanorods, gold nanofibers, silver nanoparticles, silver nanorods, silver nanofibers, platinum nanoparticles, platinum nanorods, platinum nanofibers, titania nanoparticles, titania nanorods, titania nanofibers (rutile structure, $Ti_2O_3$, $BaTiO_3$, and the like), silicon nanoparticles, silicon nanorods, silicon nanofibers, silica nanoparticles, silica nanorods, silica nanofibers, alumina nanoparticles, alumina nanorods, alumina nanofibers, calcium phosphate nanoparticles, calcium phosphate nanorods, calcium phosphate nanofibers, $BaTiO_3$ nanoparticles, $BaTiO_3$ nanorods, $BaTiO_3$ nanofibers, polycaprolactone nanofibers, polyglycolic acid nanofibers, polylactic acid nanofibers, polylacticglycolic acid nanofibers, polydoxanone nanofibers, trimethylene carbonate nanofibers, or combinations thereof. Various preferred nanomaterials are gold nanoparticles, gold nanorods, gold nanofibers, silver nanoparticles, silver nanorods, silver nanofibers, or combinations thereof.

Generally, the size of the nanomaterials are selected to be substantially similar in size to the diameter of the fibers (e.g., collagen, elastin, fibronectin, laminin, glycosaminoglycans) in the decellularized material. When collagen fibers are present in the decellularized material, the collagen fibers have a diameter of about 30 nm. In various embodiments, the nanoparticles have a mean diameter from about 5 nm to about 50 nm; from about 15 nm to about 30 nm; from about 15 nm to about 25 nm; or about 20 nm. In some of the embodiments, the nanorods, nanowires, or nanofibers have a mean diameter of from about 15 nm to about 45 nm; from about 20 nm to about 40 nm; from about 25 nm to about 35 nm; or about 30 nm. Further, the nanorods, nanowires, or nanofibers can have a mean length of from about 100 nm to about 20 $\mu$m; from about 500 nm to about 20 $\mu$m; from about 1 $\mu$m to about 10 $\mu$m; or about 10 $\mu$m.

Further, the particle sizes for the nanoparticles can be polydisperse or monodisperse. In some embodiments when gold nanoparticles are used, the nanoparticles are monodisperse. Such a diameter for the nanoparticles provides a specific surface area of from about $8.6 \times 10^4$ $cm^2/g$ to about $3.5 \times 10^5$ $cm^2/g$ ; from about $1 \times 10^5$ $cm^2/g$ to about $2 \times 10^5$ $cm^2/g$ or about $1.5 \times 10^5$ $cm^2/g$. These specific surface areas are for one nanoparticle, thus, the combined specific surface are of several nanoparticles in the bionanocomposite would be the specific surface area of one nanoparticle multiplied by the density of the nanoparticles in the bionanocomposite.

In the functionalizing step, the selected nanomaterials obtained commercially or synthesized according to various procedures in the field can be exposed to a plasma environment with selected plasma chemistry in order to introduce new functionalities which will enhance the bonding between the nanomaterials and tissue. Generally, the precursor selected for plasma polymerization is a molecule that has one or more of the desired functional groups and one or more carbon-carbon double bonds. For example, if the desired surface functional group is an amine, the precursor would contain an amine and a carbon-carbon double bond. Examples of amines that can be used in plasma polymerization are allylamine, poly(allylamine), diaminocyclohexane, 1,3-diaminopropane, heptylamine, ethylenediamine, butylamine, propargylamine, propylamine, and the like. In some embodiments, amines that can be used in plasma polymerization are poly(allylamine), diaminocyclohexane, 1,3-diaminopropane, heptylamine, ethylenediamine, butylamine, propargylamine, propylamine, and the like.

When the desired surface functional group is a carboxylic acid, the precursor would contain a carboxylic acid group and a carbon-carbon double bond. Examples of compounds used are acrylic acid, methacrylic acid, propanoic acid, and the like. When the desired surface functional group is a hydroxyl group, the precursor would contain a hydroxyl group and a carbon-carbon double bond. Examples are allyl alcohol, hydroxyethyl methacrylate, hydroxymethyl acrylate, hydroxybutyl methacrylate, and the like.

The functional groups, such as -NHx (x = 1 or 2), -OH, -COOH, are selected to act as anchoring points for crosslinking the decellularized tissue via covalent bond formation. A variety of plasma chemistry may be employed to introduce the functional groups. For example, allylamine may be used to deposit -NH, and, - $NH_2$ containing plasma coatings on the nanomaterial surfaces. Allyl alcohol, hydroxyethyl methacrylate (HEMA), acrylic acid, methacrylic acid, hydroxymethyl acrylate, hydroxybutyl methacrylate, or a combination thereof may be utilized as the monomers to deposit plasma coatings and introduce -OH, -COOH functional groups on nanomaterial surfaces. Additionaly, organosilicons including trimethylsilane (3MS) and hexa-methyldisiloxane (HMDSO) may be used to plasma coat the nanomaterials to ensure excellent adhesion of plasma coating to nanowires. The organosilicon coating provides a layer on the nanomaterial that aids adhesion of the nanoparticle to the deposited functionalized coating. Subsequent plasma treatment using $O_2$ or $CO_2$ may be used to further increase the surface concentration of these functional groups.

Furthermore, nanomaterials may be functionalized via a chemical reaction utilizing an activating agent (e.g., an agent capable of activating a carboxylic acid); for example, dicyclohexyl carbodiimide, diisopropylcarbodiimide, or ethyl dimethylaminopropylcarbodiimide. The activating agent can be used alone or in combination with an agent that improves efficiency of the reaction by stabilizing the reaction product. Once such stabilization agent is NHS (N-hydroxysuccinimide). In various embodiments, EDC (1-ethyl-3-[3-dimethylaminopropyl]carbodiimide) and NHS (N-Hydroxysuccinimide) are used as the crosslinking agents wherein EDC reacts with the carboxylic acid groups found on nanomaterials such as degradable polymers and forms an O-acrylisourea derivative and NHS stabilizes this derivative and forms a succinimidyl ester bond, which allows binding to an amino group of the tissue by forming a covalent peptide bond with the nanomaterial. When EDC and NHS are used to functionalize the nanomaterials, the molar ratio of the agents range from about 1:5 EDC:NHS to about 5:1 EDC:NHS; or about 2:5 EDC:NHS. Alternatively, nanomaterials may be functionalized via aminolysis by ethylenediamine or N-Aminoethyl-1,3-propanediamine.

For the preferred nanomaterials of gold nanoparticles, gold nanorods, gold nanofibers, silver nanoparticles, silver nanorods, silver nanofibers, or combinations thereof, the nanomaterials can be functionalized by coordinating a ligand containing the desired functional group to the gold or silver atom. Generally, the ligand should have at least two functional groups; one of the functional groups can coordinate to the metal site and the other could be used to crosslink with the decellularized material. For example, a ligand having a thiol group and an amine group; e.g., cysteine, methionine, mercaptoalkylamines such as mercaptomethylamine, mercaptoethylamine (MEA), mercaptopropylamine, mercaptobutylamine, and the like, can be coordinated to the metal of the nanomaterial to provide a functional group for further reaction with the decellularized material. Also, a ligand having a thiol group and a carboxylic acid group; e.g., thiosalicylic acid, 2-mercaptobenzoic acid, can be coordinated to the metal of the nanomaterial to provide a functional group for further reaction with the decellularized material.

When the nanomaterial is silicon carbide, the silicon carbide nanomaterial can be treated with various reagents that have at least two functional groups; one group that can react with the surface hydroxy groups on the silicon carbide and another functional group that can crosslink to the decellularized material. For example, the silicon carbide particles can be reacted with aminoalkyl-trialkoxysilanes such as aminomethyl-trimethoxysilane, aminoethyl-trimethoxysilane, aminopropyl-trimethoxysilane, aminobutyl-trimethoxysilane, aminomethyl-triethoxysilane, aminoethyl-triethoxysilane, aminopropyl-triethoxysilane, aminobutyl-triethoxysilane, aminomethyl-tripropoxysilane, aminoethyl-tripropoxysilane, aminopropyl-tripropoxysilane, aminobutyl-tripropoxysilane, aminomethyl-tributoxysilane, aminoethyl-tributoxysilane, aminopropyl-tributoxysilane, aminobutyl-tributoxysilane, or a combination thereof to provide amine groups on the surface of the silicon carbide nanomaterial.

The functionalization of the gold nanoparticles produces nanoparticles that have from about $1 \times 10^{-10}$ mol/cm$^2$ to about $1 \times 10^{-9}$ mol/cm$^2$; from about $2 \times 10^{-10}$ mol/cm$^2$ to about $1 \times 10^{-9}$ mol/cm$^2$ or from about $5 \times 10^{-10}$ mol/cm$^2$ to about $1 \times 10^{-9}$ mol/cm$^2$ functional groups per gold nanoparticle.

The decellularized tissue alone or in the bionanocomposite retains its proteins, growth factors, and other peptides. For example, the decellularized tissue retains growth factors such as vascular endothelial growth factor (VEGF), transforming growth factor (TGF-B1), proteins such as collagen, elastic, fibronectin, and laminin, and other compounds such a glycosaminoglycans. Because the decellularization process does not remove these proteins, growth factors, and other peptides, the tissue or bionanocomposite comprising the decellularized tissue can release these factors during its remodeling and resorption by the body. This release is advantageous to cell growth and cell infiltration into the affected tissue. Therefore, retention of these compounds is advantageous for the implant material.

Optionally, in addition to the endogenous proteins, growth factors, and peptides that enhance cell adhesion, cell growth, and cell infiltration into the implant material, the functionalization step may include a substep to increase tissue integration, whereas the nanomaterials may be treated with exogenous cell adhesion proteins and/or peptides. The addition of these active group will promote better cellular adhesion, vascularization, and improve overall biocompatibility. The ECM proteins are important in cell adhesion. Cell adhesion to ECM proteins is mediated by integrins. Integrins bind to specific amino acid sequences on ECM proteins such as RGD (arginine, glycine, aspartic acid) motifs.

Therefore there has been research conducted on the control of the orientation and conformation of cell adhesion proteins onto materials so that RGD motifs are accessible to integrins. For example, fibronectin and fibronectin-III have been adsorbed onto synthetic surfaces. The results showed that presence of fibronectin-III displayed more cell-binding domains than the fibronectin-free surface. Thus, it is possible to manipulate and specifically orient the cell binding proteins so that increased tissue integration is possible. Another in vivo study by Williams et al. (S.K. Williams, et al. Covalent modification of porous implants using extracellular matrix proteins to accelerate neovascularization. J Biomed Mater Res. 78A: 59-65, 2006) analyzed collagen type IV, fibronectin, and laminin type I's ability to promote peri-implant angiogenesis and neovascularization. Laminin stimulated extensive peri-implant angiogenesis and neovascularization into the porous ePTFE substrate material.

Additionally, vascular endothelial growth factor (VEGF) is a chemical signal secreted by cells to stimulate neovascularization. VEGF stimulates the proliferation of endothelial cells. TGF-B1 (transforming growth factor) is another chemical signal that stimulates the differentiation of myofibroblasts. Both types of growth factors have been incorporated into tissue engineered scaffolds to stimulate and accelerate reconstitution of native tissue.

The additional amines can be used as sites for attaching cell adhesion peptides, growth factors, glycosaminoglycans, or anti-inflammatory medications to further improve the biocompatibility of the scaffold.

Crosslinking Nanomaterial to decellularized tissue

Crosslinking of the nanomaterial to the decellularized tissue is joining the two components by a covalent bond. Crosslinking reagents are molecules that contain two or more reactive ends capable of chemically attaching to specific functional groups on proteins or other molecules (e.g., decellularized tissue). These functional groups can be amines, carboxyls, or sulfhydryls on the decellularized tissue. To react with amines in the tissue, the crosslinking agent is selected from N-hydroxysuccinimide ester (NHS ester), N-gamma-maleimidobutyryloxy succinimde (GMBS), imidoester (e.g., dimethyl adipimidate, dimethyl pimelimidate, dimethylsuberimidate, dimethyl 3,3'-dithiobispropionimidate•2 HCl (DTBP)), pentafluorophenol ester (PFP ester), hydroxymethyl phosphine. A carboxyl group on the tissue can react with an amine on the nanoparticle directly by activation with carbodiimide. Various carbodiimides can be used including 1-ethyl-3-(3-dimethylaminopropyl)carbodiimide, dicyclohexyl carbodiimide, diisopropylcarbodiimide, and the like. A sulfhydryl group on the tissue can react with a malemide (e.g., N-e-Maleimidocaproic acid (EMCA)), haloacetyl (e.g., SBAP (NHS ester/bromoacetyl), SIA (NHS ester/iodoacetyl), SIAB (NHS ester/iodoacetyl), Sulfo-SIAB (sulfo-NHS ester/iodoacetyl), , pyridyldisulfide (1,4-di(3'-(2'-pyridyldithio)-propionamido)butane (DPDPB), sulfosuccinimidy 6-(3'-[2-pyridyldithio]-propionamido)hexanoate (Sulfo-LC-SPDP), N-[4-(p-azidosalicylamido)butyl]-3'-(2'-pyridyldithio)propionamide (APDP)), or vinyl sulfone.

To enhance the crosslinking between the selected nanomaterials and decellularized tissue, the functionalized nanomaterials with surface functional groups capable of bonding with tissue are preferred over the "naked" nanomaterials. Though a variety of functional groups may be selected, according to one embodiment of the invention various functional groups that are capable of forming covalent peptide bonding with tissue, such as -NH, -NH$_2$, -COOH, or a combination thereof, are employed.

In the crosslinking step, depending on the surface functional groups introduced, the functionalized nanomaterials are incubated (or mixed) with the decellularized tissues in a crosslinking solution via a crosslinking procedure available or known to the researchers in the field. In some embodiments, the crosslinking agent can be N-gamma-maleimidobutyryloxy succinimde (GMBS), N-e-Maleimidocaproic acid (EMCA), and Dimethyl 3,3'-dithiobispropionimidate•2 HCl (DTBP). For example, according to one embodiment, the crosslinking solution may contain acetone, 1x PBS (phosphate buffered saline), EDC (1-ethyl-3-[3-dimethylaminopropyl]carbodiimide) and NHS (N-Hydroxysuccinimide). For the crosslinking reaction, a tissue:solution volume ratio of from about 1:100 to about 20:100; from about 5:100 to about 15:100; from about 7:100 to about 10:100; or an 8:100 ratio is maintained and for rinsing, a tissue:solution volume ratio from about 0.1:100 to about 10:100; from about 0.5:100 to about 2:100; or 1:100 ratio is maintained for all subsequent rinses.

Various concentrations of nanomaterials may be utilized to achieve optimal crosslinking. The incubation generally lasts about 24 hours at room temperature on an orbital shaker table at low rpm. Following incubation, the resulting crosslinked tissues are vigorously rinsed with 1xPBS for 48 hours on an orbital shaker table with several changes of the PBS solution to remove residual crosslinkers and unbound nanomaterials. Crosslinked tissues are then stored in 1x PBS at 4 °C until subsequent testing or sterilization occurs.

The crosslinking density in the bionanocomposite can generally be measured by a collagenase assay wherein an increase in release of hydroxyproline indicates degradation of collagen. It would be expected that tissues that had lower crosslinking density would have a greater rate of collagen degradation and result in more hydroxyproline being released. Further, the mechanical properties can measure the crosslinking density wherein the tensile strength would be expected to increase with increasing crosslinking density. Further, the differential scanning calorimetry measurements indicate the crosslinking density of the material because a material that has a greater crosslinking density should have a higher denaturation temperature.

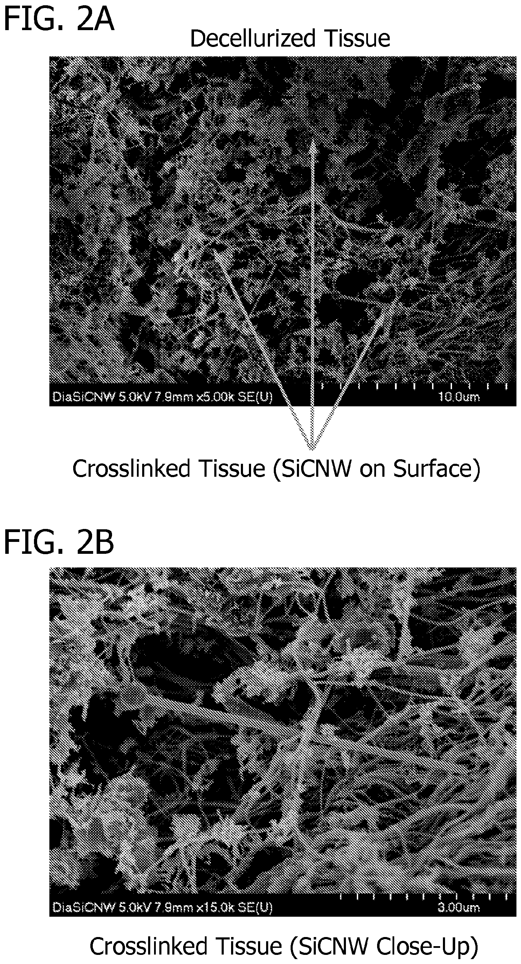

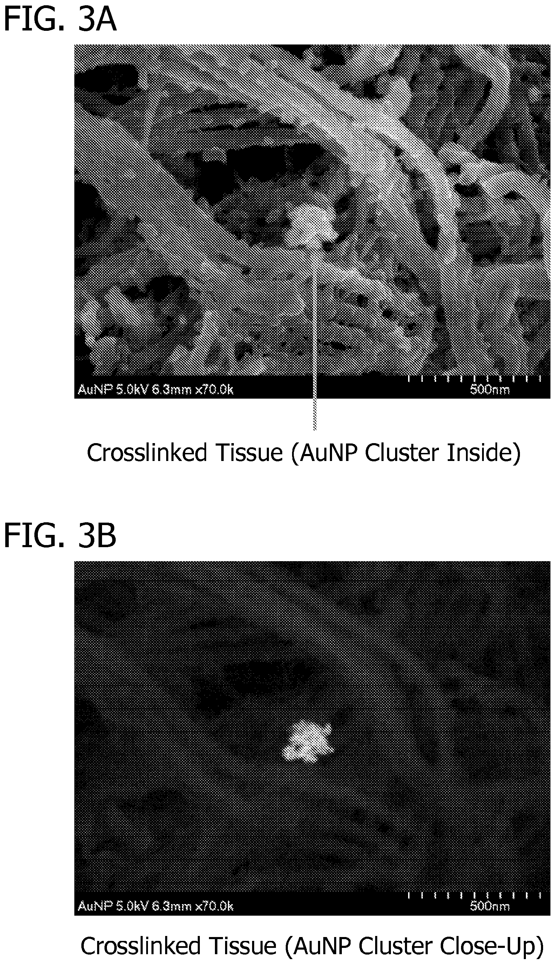

SEMs of the Au-nanoparticles crosslinked with decellularized porcine diaphragm tendon tissue and silicon carbide nanowires crosslinked with decellularized porcine diaphragm tendon were obtained. Figures 2A-B are the SEMs of Au-nanoparticles crosslinked with decellularized porcine diaphragm tendon tissue. Figures 3A-B are the SEMs of two sets of silicon carbide nanowire crosslinked with decellularized porcine diaphragm tendon tissue.

Bionanocomposites

The mechanical and chemical properties of the bionanocomposites desirably do not change significantly once implanted in an animal. For example, the viscoeslasticity of the bionanocomposite does not change significantly as cells from the surrounding tissue infiltrate the bionanocomposite and it degrades. In order to have a composite that has a desired viscoelasticity, the material should have an appropriate degradation rate. Further, the viscoelasticity can be measured by the Young's modulus wherein a higher value means the material is stiffer and a lower value means the material is less stiff. Preferably, the viscoelasticity of the bionanocomposite is from about 100 MPa to about 200 MPa; from about 110 MPa to about 200 MPa; from about 110 MPa to about 190 MPa; or from about 110 MPa to about 180 MPa.

The bionanocomposites can have a range of geometries depending on the desired use. For example, the decellularized tissue can be cut to fit the particular site either before or after crosslinking to the nanoparticles. Thus, the bionanocomposite can be a range of dimensions and shapes. For example, the bionanocomposite can be a regular or an irregular shape, namely, a square, rectangle, trapezoid, parallelogram, triangle, circle, ellipsoid, barbell, or any irregular shape that is appropriate to the use thereof.

Further, the nanoparticles, nanowires, nanofibers, or nanorods can be distributed uniformly on the surface and/or within the decellularized tissue. In other embodiments, the nanoparticles, nanowires, nanofibers, or nanorods can be distributed nonuniformly on the surface and/or within the decellularized tissue. The density of the nanoparticles on the surface of the decellularized surface and/or within the decellularized tissue can be optimized to provide the appropriate surface area for cell growth, infiltration, and vascularization. When nanoparticles are used that have a mean diameter of from about 15 nm to about 30 nm, preferably 20 nm, the nanoparticles can infiltrate into the decellularized tissue and provide a surface for cell growth. When nanowires, nanofibers, or nanorods having a diameter of about 20 nm to about 30 nm are used, the degree of infiltration of these materials into the decellularized material depends on the length of the nanowire, nanofiber, or nanorod. If the nanowire, nanofiber, or nanorod is too long, it cannot infiltrate into the decellularized tissue.

Depending on the chemical identity of the nanoparticles that are crosslinked to the decellularized tissue, the bionanocomposite can scavenge free radicals. For example, gold nanoparticles, gold nanorods, and gold nanofibers have the ability to scavenge free radicals. Without being bound by theory, it is believed that the free radical scavenging ability of the gold nanoparticles is able to ameliorate and/or reduce inflammation at the bionanocomposite implant site as shown in example 2. The free radical scavenging capability of the gold nanoparticle bionanocomposite can be measured using the technique of Hsu et al., J. Biomedical Materials Research Part A 2006, 759. The capacity of the sample to scavenge can be measured by placing the sample (7.5 mm diameter, 1 mm thick) in 3 mL of 32 $\mu$M 2,2-diphenyl-1-picrylhydrazyl (DPPH), vortexed, and left to stand at room temperature for 90 minutes. The absorbance of the reaction mixture can be measured at 515 nm using a UV/VIS spectrophotometer and the following equation:

$$\text{Scavenging ratio (\%)} = [1 - \text{Absorbance of test sample/Absorbance of control}] \times 100\%.$$

Thus, the free radical scavenging ratio of the gold nanoparticle bionanocomposite is expected to be higher than the scavenging ratio of the decellularized material without gold nanoparticles.

When the bionanocomposite is implanted at a desired site in an animal. There is typically an underlying layer of muscle, then the bionanocomposite implant and an overlying layer of tissue. Thus, immediately after the placement of the implant until the time that the implant has been completely absorbed by the body, these three layers will be present. Over time, the overlying tissue will migrate and infiltrate the implant and the border between the implant and the tissue will be compromised.

The biodegradability of the implant is usually determined by removing the implant and surrounding tissue from the animal and performing a visual inspection of the margins between the underlying muscle and the implant as well as the overlying tissue and the implant. At a certain time after placement, the margin between the tissue (muscle or other tissue) and the implant will not be visible. At this point the implant in considered to be completely biodegraded. Preferably, the time for complete degradation of the implant is substantially the same as the healing time for the tissue. For example, the time for degradation ranges from about 1 month to about 12 months; from about 1 month to about 9 months; from about 1 month to about 6 months; from about 2 months to about 6 months; or from about 3 months to about 6 months.

The biocompatibility, mechanical properties, and *in vivo* stability of the bionanocomposite render it suitable for use in hernia repair, meniscus tissue replacement, and vascular grafts. The composite has a supple, flexible membranous structure substantially similar to the intact biologic material from which it is produced. It is resilient so that it can be rolled, stretched or otherwise deformed in use, e.g., in the course of surgical implantation and revert to its original configuration when external forces holding the composite in the deformed configuration are removed. For example, a substantially planar bionanocomposite useful in hernia repair possesses a springiness which allows it to be rolled into a tightly coiled configuration for insertion through a laproscopic incision and then revert to its planar configuration inside the abdominal cavity when it is no longer held in the coiled configuration. This facilitates use of laproscopic surgical techniques to implant the composite in a subject so that it can function in reinforcement of the abdominal wall during and after convalescence from the surgery. As used herein, "substantially planar" means the bionanocomposite can have an irregular surface and be somewhat curved.

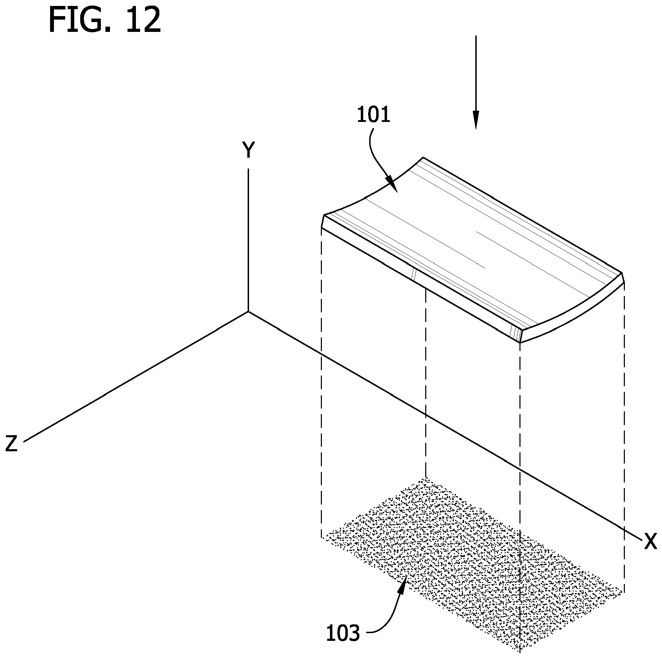

Especially important to the function of the bionanocomposite is its stability *in vivo*. It retains its suppleness and flexibility during healing of the surgical site at which is installed and indefinitely thereafter until it has been integrated with surrounding tissue, or infiltrated and effectively displaced by indigenous tissue. The implanted bionanocomposite is resistant to oxidation, and resistant to shrinkage and/or hardening. For example, after passage of 30, 60 or 90 days following surgery, the area occupied by a projection of a substantially planar membranous bionanocomposite used in hernia repair on a plane generally parallel to a plane of best fit (e.g., as determined using the least squares method) to the bionanocomposite remains at least 75%, more typically at least 80%, most typically at least 90% of the area occupied by a comparable projection of the composite prior to implantation. Figure 12 illustrates projection 103 of a bionanocomposite 101 onto a surface that is parallel to a plane of best fit (not shown) to the bionanocomposite. The Young's modulus and flexural modulus of the bionanocomposite each remain between 50% and 200%, more typically between 75% and 150%, most typically between 90% and 125% of their values prior to implantation after passage of 30, 60 and 90 days.

After 3 months, 6 months, 9 months or one year after implantation or until the biocomposite is effectively displaced by endogenous tissue, the above defined projected area remains at least 60%, more typically, at least 75%, most typically at least 90% of the comparable projected area prior to implantation, and the Young's modulus and flexural modulus each remain between 50% and 250%, more typically between 75% and 200%, most typically between 90% and 150%, of their values prior to implantation.

Synthesis

Also described is a method for fabricating the Bionanocomposite.
The inventive method includes three major steps 1) decellularizing a piece of pre-selected biological (may also be called natural) tissue, 2) functionalizing a pre-selected nanomaterials, and 3) crosslinking the decellularized tissue with the functionalized nanomaterials.

The decellularizing step may include a substep of selecting a piece of biological tissue, which may be obtained commercially, or harvested via either allografts or xenografts. The selected natural tissue may be cut into the desired shapes and sizes and needs to be stored in a buffered solution containing protease inhibitors and bacteriostatic agents at pH about 8 and 4°C to prevent degradation of the tissue by lysosomal enzymes released by the biological cells.

Uses

The inventive Bionanocomposite may be used in a wide range of tissue engineering applications, where the Bionanocomposite is made into scaffolds to repair defective tissue or to deliver cells, growth factors, and other additives to a healing site. For example, the Bionanocomposite can be utilized as a soft tissue repair material for such applications as hernia repair, meniscus tissue replacement, and vascular grafts.

Preliminary testing indicates that Bionanocomposite materials possess adequate mechanical properties for many soft tissue repair applications. For example, Bionanocomposites crosslinked with gold nanoparticles or silicon carbide nanowires have a tensile strength (at yield) of 19.50±2.1 MPa and 20.54±1.0 MPa respectively by standard tensile testing. In comparison, the decellularized porcine small intestine submucosa tissue (commercially available, Surgisis Gold, Cook Biotech Inc., West Lafayette, IN), which is commercially available and currently used in tissue repair, has a tensile strength (at yield) of 17.48±2.2 MPa under the identical test conditions. The detailed test protocol is described in the example section. Another commercially available acellular porcine dermal mesh (Permacol, TSL, Aldershot, Hampshire, England), which is crosslinked with hexamethylene diisocyanate, has a mean tensile strength of 21 ± 6MPa according to the company data. (http://www.tissuescience.com/sitecontent/corporate.htm) Thus, Bionanocomposite materials possess similar mechanical strength as tissue-derived materials already in use for soft tissue repair applications.

The testing results (discussed in detail in the example section) also show that the decellularized tissue crosslinked with nanomaterials provides improved biocompatibility over the naked decellularized tissue. The decellularized tissue crosslinked with nanomaterials when implanted also favorably affects cellular responses.

Having described the invention in detail, it will be apparent that modifications and variations are possible without departing from the scope of the invention defined in the appended claims.

**EXAMPLES**

The following non-limiting examples are provided to further illustrate the present invention and further provides several examples of the inventive method of fabrication of the Bionanocomposite and the testing thereof.

Example 1: Decellularized porcine diaphragm tendon crosslinked with AuNP or SiCNW

Decellularization of central tendon portion of porcine diaphragm. When selecting and storing natural tissue, the surrounding muscle of the porcine diaphragm is first removed so that only the collagen rich central tendon portion remains. The resulting natural tissue is placed immediately into Tris buffer solution (pH 8.0) containing 5 mM ethylenediaminetetraacetic acid (EDTA), 0.4 mM phenylmethanesulfonyl fluoride (PMSF), and 0.2% (w/v) sodium azide and stored at 4 °C until ready to use.

In the decellularization step, a 7 cm x 7cm piece of natural tissue is placed into Tris buffer solution (pH 8.0) containing 5 mM ethylenediaminetetraacetic acid (EDTA), 0.4 mM phenylmethanesulfonyl fluoride (PMSF), 0.2% (w/v) sodium azide, and 1% (v/v) tri-n-butyl phosphate (TnBP). The reaction is placed on a shaker table at room temperature for 24 hours at 225 rpm.

In the rinsing step, the resulting tissue is rinsed with deionized water on shaker table at room temperature for 24 hours at 225 rpm, then with 70% (v/v) ethanol on shaker table at room temperature for 24 hours at 225 rpm. The resulting decellularized tissue as shown in Figure 1B indicates a normal collagen architecture showing that the characteristic collagen banding pattern remained undisturbed after decellularization treatment, and an acellular collagen scaffold has been achieved.

Fabrication of gold nanoparticle or silicon carbide nanowire crosslinked with pig diaphragm tendon tissue [Au-bionanocomposite and SiC-bionanocomposite]. The gold nanoparticles were functionalized with amine groups via L-cysteine (Sigma Aldrich) by combining equal volumes of gold colloid solution with 55 $\mu$g/mL aqueous cysteine solution, and the silicon carbide nanowires were functionalized with amine groups via plasma treatment with an allylamine monomer. The crosslinking solution was comprised of a 50:50 (v/v) solution of acetone and 1x phosphate buffered saline (PBS) (pH 7.5) with a final concentration of 2 mM EDC and 5 mM NHS. The NHS was initially dissolved in a small volume of dimethylformamide (DMF), and the EDC was likewise dissolved in a small volume of 0.1M MES (2-(N-Morpholino)ethanesulfonic acid) with 0.5M NaCl (pH 6.0). The two solutions were immediately mixed together and added to the acetone/PBS solution. The decellularized tissues were reacted with this crosslinking solution at room temperature for 15 minutes to activate the carboxyl groups present on the collagen molecules. After this incubation period, the amine-functionalized nanomaterials were added at the following concentrations: 1 mL gold nanoparticle solution per 100 mL of crosslinking solution or 1 mg silicon carbide nanowires per 1 mL of crosslinking solution, and 3.0mL of 15$\mu$M mercaptoethylamine (MEA)-functionalized AuNP. All tissues were allowed to incubate at room temperature for 24 hours with constant agitation, followed by 48 hours of rinses with 1xPBS in which the PBS was changed after 24 hours. As shown in FIGs 2 and 3, the nanomaterials integrated into the decellularized tissue to form a bionanocomposite.

Preparation of mercaptoethylamine functionalized gold nanoparticles. Gold nanoparticles (20nm diameter) were purchased from RDI Division of Fitzgerald Industries International (Concord, MA) in the form of a gold colloid solution. The AuNP were then functionalized with $\beta$-mercaptoethylamine hydrochloride (MEA) from MP Biomedicals (Solon, OH) in order to functionalize them with terminal amine groups to promote covalent bonding to the porcine diaphragm tendon.

The optimal concentration of MEA was determined through the use of ultraviolet-visible spectroscopy and a protocol found in the literature by Bellino et al. (Bellino MG, et al. Physical Chemistry Chemical Physics 2004;6:424-428.) Briefly, a Beckman DU520 UV-Vis Spectrophotometer (Beckman Instruments, Inc., Fullerton, CA) was utilized to acquire the spectrum of plain AuNP without any additives. Subsequent scans were performed after successive 3.33$\mu$L additions of an aqueous 0.4mM $\beta$-mercaptoethylamine (MEA) solution (MP Biomedicals, Santa Ana, CA). The MEA concentrations evaluated during this process ranged from 1.3$\mu$M to 23.8$\mu$M, and the optimal concentration was defined as the concentration at which the absorbance value at 525nm remained constant even with further increasing the MEA concentration. Ultimately, 15$\mu$M MEA was chosen as the optimal concentration to be utilized to functionalize the AuNP in this study.

Tensile Test Characterization. Four pieces of each type of bionanocomposite material (~15 mm x 52 mm) were notched on both sides to reduce the width of the tissue by 50%. This created a stress concentration at the center of the specimen and prevented failure of the tissue at the grips. The tissues were gripped at each end with 20 mm x 34 mm waterproof sandpaper-coated grips, and a Texture Analyzer (TA.XT2) was utilized at a strain rate of 0.2 mm/s until failure. The tensile strength of each tissue (at yield), $\sigma_u$, was calculated by dividing the maximum load, $F_{max}$, by the

original cross-sectional area, A, of the specimen.

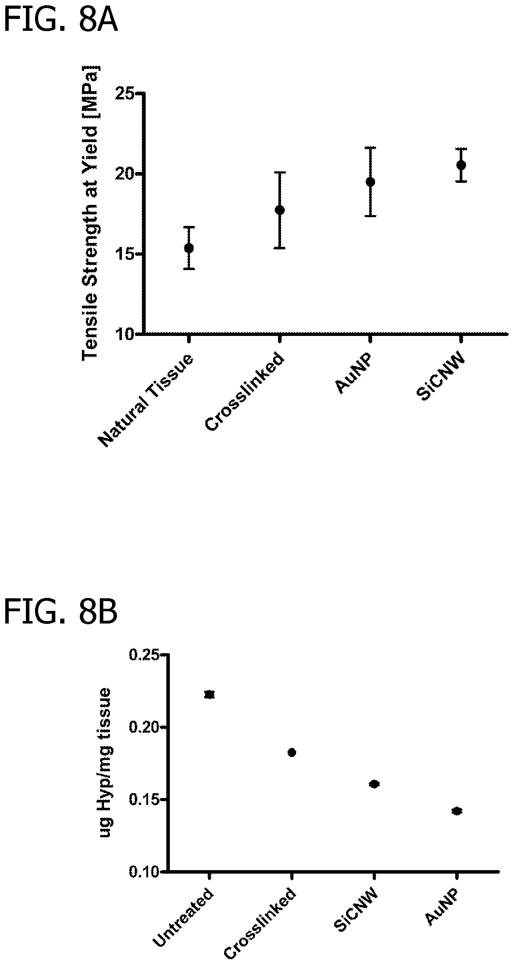

Four types of tissue, the porcine diaphragm tendon tissue (Natural Tissue), the decellularized porcine diaphragm tendon tissue (Decellularized Tissue), the Au-nanoparticle crosslinked decellularized porcine diaphragm tendon tissue (AuNP), and the SiC-nanowire crosslinked decellularized porcine diaphragm tendon tissue (SiCNW), were tested via the above mentioned procedure. The tensile data showed a 32.5% (SiC nanowires, $20.5 \pm 1.0$ MPa) and 26.6% (AuNPs, $19.5 \pm 2.1$ MPa) increase in the tensile strength of the bio-nanocomposite as compared to the ECM scaffolds without nanomaterials (natural tissue, $15.4 \pm 1.3$ MPa). The tensile test results are shown in Figure 8A.

Collagenase assay. A collagenase assay was performed according to the method described by Duan and Sheardown. (Duan X, et al. Journal of Biomedical Materials Research Part A 2005;75:510-518) Briefly, five samples of each of the four types of tissues were dehydrated at ambient temperature for 24 hours. Approximately 5mg of each tissue were then incubated for 1 hour at 37°C in 1.0mL of 0.1M Tris buffer containing 0.05M $CaCl_2$ (pH 7.4). After this incubation, 200 Units of bacterial collagenase (Clostridium histolyticum, Sigma Aldrich) were added along with another 1.0mL of the same Tris buffer. The tissues were incubated for 24 hours at 37°C until the reaction was stopped by the addition of 0.2mL of 0.25M EDTA and the mixture cooled on ice for 10 minutes. Each sample was centrifuged at 3000g for 15 minutes, and $40\mu L$ of supernatant was combined with $160\mu L$ of 2.SN NaOH and autoclaved at 120°C for 40 minutes. Hydroxyproline standards and a blank containing $0\mu g$ hydroxyproline were also subjected to the same treatment. After the samples were hydrolyzed by autoclaving, 1.8mL of 0.056M chloramine-T solution was added to each sample and reacted at ambient temperature for 25 minutes. Then 2.0mL of 1.0M Ehrlich's reagent (p-dimethylaminobenzaldehyde) dissolved in a 2:1 solution of propanol and perchloric acid was added to each sample and reacted at 65°C for 20 minutes. The absorbance was read on a Beckman DU520 UV-Vis Spectrophotometer (Beckman Instruments Inc., Fullerton, CA) at 550nm. The $\mu g$ of hydroxyproline released from each sample after digestion by collagenase was calculated based on the standard hydroxyproline curve. This value was divided by the original mass of the tissue to yield the $\mu g$ of hydroxyproline released per mg of original tissue. Fifteen measurements were taken for each type of tissue (n=15). The results of the study are detailed in Figure 8B.

Flow Cytometry. Four types of tissue, the porcine diaphragm tendon tissue (Natural Tissue), the decellularized porcine diaphragm tendon tissue (Decellularized Tissue), the Au-nanoparticle crosslinked decellularized porcine diaphragm tendon tissue (AuNP), and the SiC-nanowire crosslinked decellularized porcine diaphragm tendon tissue (SiCNW), were each cut into three circular pieces (1 cm diameter) and sterilized by an aqueous solution of 0.1 % (v/v) peracetic acid and 1 mM NaCl at room temperature for 24 hours with constant agitation, followed by a 24 hour rinse with sterile 1x PBS. The tissues were then incubated overnight in sterile Eagle's Minimum Essential Media containing 10% horse serum and 200 U/mL PenStrep at 4 °C. Each piece of tissue was then placed in a separate well of a 6-well tissue culture plate and seeded with 120,000 L929 murine fibroblast cells suspended in 6 mL of sterile media containing 10% horse serum and 200 U/mL PenStrep. Similarly, control cells were seeded with the same density in empty wells of a 6-well tissue culture plate. All cells were allowed to incubate at 37°C with 5% $CO_2$ for 3 days, after which they were stained with propidium iodide (PI) according to the instructions from Cell Technology, Inc. PI is a fluorescent dye that cannot permeate the membranes of normal, viable cells. In necrotic or membrane-compromised cells, however, PI intercalates with the cell's DNA, thus it was utilized in this study to stain "non-vital" cells. A FACScan (Becton Dickinson) flow cytometer was utilized to acquire the fluorescent signal and differentiate between the number of live cells versus dead cells in each treatment group. All flow cytometry experiments were repeated three times (n=3).

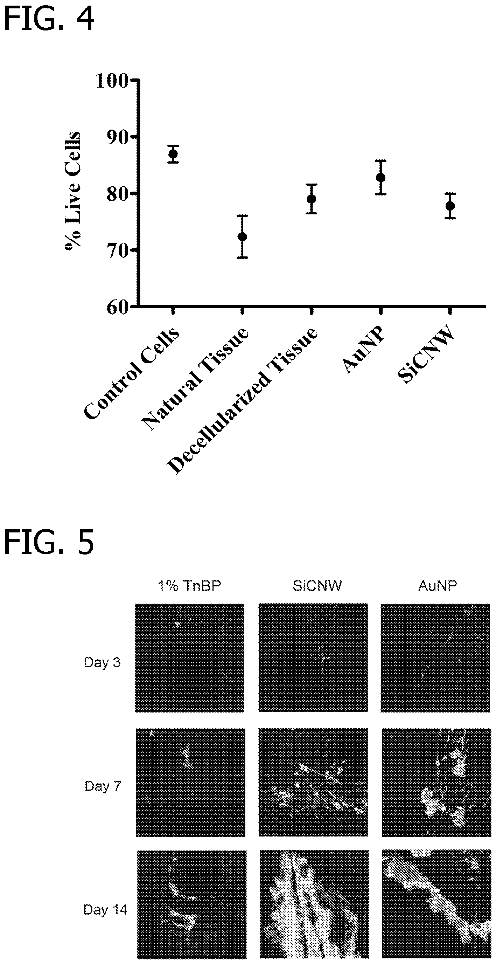

AuNP and SiCNW were tested against Natural Tissue and Decellularized Tissue, and the results are shown in FIG. 4. In FIG. 4, the number of "live" cells in contact with decellularized tissue versus nanomaterial-decellularized tissues was very similar, indicating low cytotoxicity of nanomaterials when bound to decellularized tissue. Flow cytometry results for the Natural Tissue indicated that 72% (mean) of cells remained viable after three days in contact with tissue that had not been decellularized. This represents a significant amount of cell death (p<0.05) relative to the control cells, which had a mean viability of 87% after harvesting and processing for flow cytometry. The results also indicated that decellularizing the tissue with 1%TnBP and crosslinking with SiC or AuNP improved its biocompatibility and resulted in 79%, 78%, and 83% mean viability respectively.

Bioreactor Tests. The purpose of the study was to determine if the nanomaterial- crosslinked-ECMs can favorably affect cellular responses. Three groups of tissues were examined: AuNP, SiC, and Decellularized Tissue (treated with 1% TnBP). The tests examined cellular viability, cellular distribution, cellular content, collagen content and GAG content. For the study, synovial intima was harvested from dogs humanely euthanized for reasons unrelated to this study. Synovial fibroblasts were cultured from the intima following a procedure by Cook and Fox. (Cook JL, et al. American Journal of Veterinary Research 2008;69:148-156)

Scaffold preparation and construct culture. Fifteen (n=15) disks from each group were placed in individual wells of 6 well culture plates in DMEM+FBS for 24 hours, placed in sterile incubators at 37°C, 5% CO2, 95% humidity as a pre-soaking conditioning. After pre-soaking, media was removed from each well and replaced with the fibroblast cell solution at a concentration of $1X10^6$ cells/ml. Plates were agitated for 24 hours. Following the cell-loading, each construct was placed into one of three 110 ml rotating bioreactor flasks. The flasks were rotated at ~50 rpm. Media changes were completed every third day by replacing 50% of the volume of DMEM+FBS.

Construct harvest and assessment. Five (n=5) constructs were harvested from each group at day 3, 7 and 14. Cross-sections were taken from each disk for cellular viability and distribution assessment. Cell viability was determined with the use of ethidium homodimer-1 and Calcein AM fluorescent stains and the use of Confocal Laser Microscopy. One mm sections were made and incubated with the staining agents for 30 minutes, placed on a glass microscope slide, moistened with several drops of PBS, 1X, and stained using the fluorescent double labeling technique. The sections were examined under 10x magnification. Live and dead cell counts were determined using digital image analysis using the stored images via a threshold algorithm and color filter analysis. Two additional cross sections were harvested from each disk, formalin-fixed and paraffin embedded and stained with H&E for cellular distribution analysis. Images were captured at 10X and regional cell counts (peripheral versus central) completed via digital image analysis. The remainder of each construct was lyophilized, and a dry weight obtained, and then mixed with 1ml Papain Solution. Portions of each digest were used to determine GAG content by the dimethylmethylene blue assays, and collagen content by determining hydroxyproline concentrations. The remaining solution was incubated at 600C in a water bath for 4 hours. The Quant-iT PicoGreen™ double stranded DNA quantification assay (Invitrogen) was used to determine the cellularity of the remaining scaffold. Double stranded DNA extracted from bovine thymus was mixed with TE buffer (Invitrogen) to create standard DNA concentrations of 1,000, 100, 10, and 1 ng/mL. The standards and 100uL of each papain digested sample (in the above GAG and hydroxyproline assays) were added to a 96 well plate. 100uL of 2ug/mL of Pico Green reagent was added to each well and incubated for 5 minutes. Sample fluorescence was read by a plate reader (BioTec). Absorbances were converted to ng/mL concentrations and total double stranded DNA yield in ng using FT4 software.

Cell viability and distribution are described subjectively. Differences in DNA, collagen and GAG content were determined using a one-way repeated measures ANOVA followed by a Tukey all pair-wise multiple comparison test with significance set at $p < 0.05$. Collagen and GAG contents were normalized for DNA content to eliminate cellularity as a source of differing concentrations.

Cell Viability Results. Because of cellular clumping on the scaffolds, digital image analysis was not able to be utilized to determine percent viability. Cell viability ranged from 0% to 100% in areas of all groups, however percent viability increased to consistently greater than 90% in all groups over time. In Day 14, rows of highly proliferative and viable cells can be seen aligning themselves with the nanomaterials as shown in FIG. 5.

Cell Distribution Results: Cells were able to penetrate internally into scaffolds of all groups visible from Day 3. In the SiC group, cells associated with visible SiC nanowires could be detected. No evidence of AuNPs was visible histologically. Day 7 demonstrated more robust internalization of larger rafts of cells into areas of loosely bundled collagen fibers and around voids left by the SiC nanowires. By Day 14, cells in all groups were proliferating between more tightly associated collagen fibers as the cellular integration was becoming more complete.

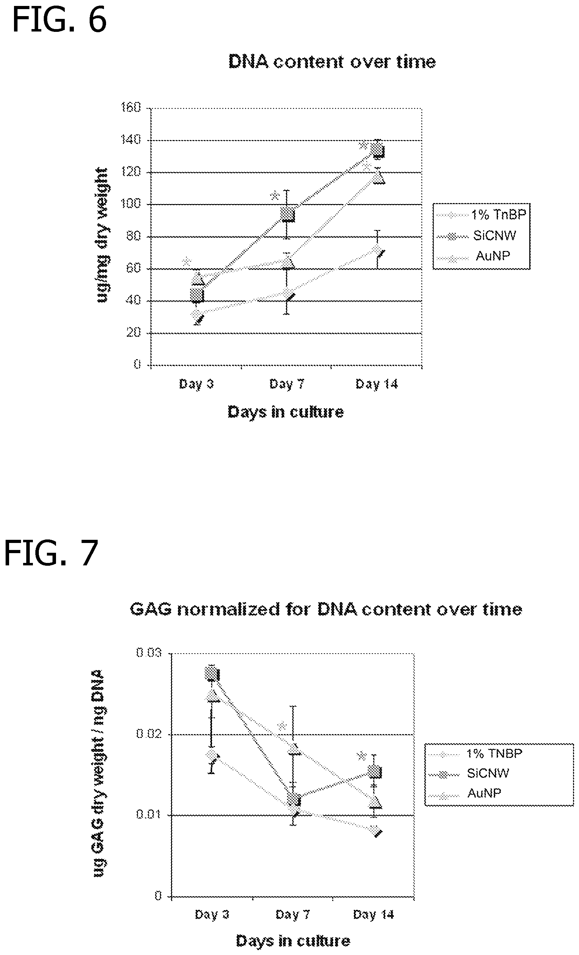

Cellular content (DNA quantification) Results. As shown in FIG. 6, by day 3, the AuNP group had significantly more DNA per dry weight than the control (1%TnBP) group ($p = 0.029$). By Day 7, the SiC group possessed more DNA than the 1%TnBP group ($p = 0.011$), and by day 14, both SiC and AuNP groups possessed a higher concentration of doublestranded DNA per dry weight than the control ($p = 0.007$ and $0.039$ respectively).

Collagen content Results. On day 7, AuNP showed higher collagen than the 1%TnBP group ($p = 0.018$) and by day 14 the SiC group possessed higher total collagen than 1%TnBP ($p = 0.014$). However when collagen contents per dry weight were normalized for DNA content to determine the effects of increasing cellularity on collagen production, no significant differences were detected between groups at any time point.

GAG Content Results. On day 3, significant differences were detected in total GAG content among all three groups, with AuNP exhibiting the highest amount and the control group possessed the least amount. By day 7, both nanomaterials groups were producing more GAG than the control and on Day 14, only the SiC group had concentrations significantly higher than the other groups. When this data was normalized for DNA content to determine the effects of cellularity, differences were noted on day 7, where the AuNP possessed more GAG than other groups and on day 14 where SiC exhibited higher concentrations of GAG than the control group. Over time, all groups demonstrated declining production of GAG as cellularity increased as shown in FIG. 7.

Treatment of porcine diaphragm central tendon with SiC nanowires or AuNPs appeared to enhance cellularity of the scaffolds *in vitro*. Based on histologic and laser microscopy imaging, the nanowires and nanoparticles may establish conduits and cavities upon which the cells may grow and extend deeper into the tightly intermeshed collagen matrix of the central tendon tissue, thus optimizing early cellular infiltration, protection and potentially mitogenesis. The rise in cellularity of the treated scaffolds resulted in more net production of hydroxyproline, used here as a marker of collagen deposition. There was no direct effect of the treated scaffolds on collagen production, however. Interestingly, GAG content decreased over time in all groups, but tended to decrease less in those scaffolds treated with the silicon carbide and gold at various times. The reason for the decrease in GAG is unknown. Synovial fibroblasts can produce glycoproteins naturally as part of their extracellular matrix. However, without maintaining appropriate bioactive signaling, this production may be reprioritized in light of more important cellular functions when placed in a new environment, such as cellular migration, proliferation and collagen production.

The testing results showed that both SiC nanowire and AuNPs treatment of porcine diaphragm central tendon appear to optimize properties associated with early cellularization and some components of extracellular matrix formation by synovial fibroblasts.

Example 2: *In vivo* implant study in rats

Experimental design. Forty-five male, Sprague-Dawley rats were divided into the following five treatment groups. Fifteen rats were sacrificed at each of the three time points (seven, twenty-one, and ninety-seven days). The abdominal walls of the rats and any remaining scaffold materials were recovered at these times and subjected to histological analysis to determine whether differences existed between the inflammatory response to the scaffolds, fibroblast infiltration, and neovascularization.

"Control" rats treatment group (one per time point): Rats in this treatment group did not undergo surgery, so tissues recovered from these animals served as examples of normal, healthy abdominal wall. "Sham" rats treatment group (two per time point): Rats in this treatment group underwent the surgical procedure but no scaffold materials were implanted. "Decellularized" scaffolds treatment group (four per time point): Rats in this treatment group received an implant comprised of porcine diaphragm tissue that was subjected to the decellularization process and subsequently sterilized with peracetic acid. "AuNP" bionanocomposite scaffolds treatment group (four per time point): Rats in this treatment group received an implant comprised of porcine diaphragm tissue that was decellularized, crosslinked with amine-functionalized gold nanoparticles (AuNP) in combination with EDC and NHS, and subsequently sterilized with peracetic acid. "SiCNW" bionanocomposite scaffolds treatment group (four per time point): Rats in this treatment group received an implant comprised of porcine diaphragm tissue that was decellularized, crosslinked with amine-functionalized silicon carbide nanowires (SiCNW) in combination with EDC and NHS, and subsequently sterilized with peracetic acid.

Preparation of scaffolds. Porcine diaphragms were harvested from the University of Missouri abattoir within four hours of euthanasia. The central tendon portion of the diaphragm was dissected from the surrounding muscle and immediately immersed in a Tris buffer solution (pH 8.0) containing 5mM ethylenediaminetetraacetic acid (EDTA), 0.4 mM phenylmethylsulfonyl fluoride (PMSF), and 0.2%(w/v) sodium azide and stored at 4°C. The porcine tendons were decellularized as described in example 1.

Crosslinking. Following decellularization, the porcine tendons were crosslinked utilizing either amine-functionalized silicon carbide nanowires (SiCNW) or amine-functionalized gold nanoparticles (AuNP) in conjunction with 1-ethyl-3-[3-dimethylaminopropyl]carbodiimide (EDC) and N-Hydroxysuccinimide (NHS).

The amine-functionalized SiCNW (30nm in diameter and approximately 10μm in length) were generously provided by Andrew Ritts, Dr. Qingsong Yu, and Dr. Hao Li (University of Missouri, College of Engineering, Department of Mechanical & Aerospace Engineering, Columbia, MO). The SiCNW were synthesized via chemical vapor deposition and subsequently functionalized with amine functional groups through plasma treatment with an allylamine monomer.

The gold nanoparticles (20nm diameter) were purchased from RDI Division of Fitzgerald Industries International (Concord, MA) in the form of a gold colloid solution. The AuNP were functionalized with L-cysteine (Sigma Aldrich) by combining equal volumes of gold colloid solution with 55μg/mL aqueous cysteine solution.

The crosslinking solution was comprised of a 50:50(v/v) solution of acetone and 1x phosphate buffered saline (PBS) (pH 7.4) with a final concentration of 2mM EDC and 5mM NHS. The NHS was initially dissolved in 1.0mL of dimethylformamide (DMF), and the EDC was likewise dissolved in 1.0mL of 0.1M 2-(N-Morpholino)ethanesulfonic acid (MES) with 0.5M sodium chloride (NaCl) (pH 6.0). The two solutions were immediately mixed together and subsequently added to the acetone/PBS solution. The tissues were reacted with this solution at room temperature for 15 minutes to activate the carboxyl groups present on the collagen molecules. After this incubation period, the amine-functionalized nanomaterials were added at the following concentrations: 1.0mL AuNP solution per 100mL of crosslinking solution or 50mg SiCNW per 100mL of crosslinking solution. The tissues were incubated in this solution for 24 hours with gentle agitation at room temperature for 24 hours. This treatment was followed by 48 hours of rinses with 1x PBS with constant agitation, in which the PBS was changed after 24 hours.

Sterilization. Prior to surgical implantation, all three types of scaffolds were cut into 1cm$^2$ pieces and sterilized. Sterilization was achieved by incubation in an aqueous solution of 0.1%(v/v) peracetic acid and 1.0M NaCl at room temperature for 24 hours with constant agitation. This treatment was followed by 48 hours of rinses with sterile, 1x PBS in which the PBS was changed after 24 hours. The scaffolds were then incubated overnight in 70%(v/v) ethyl alcohol at 4°C and surgically implanted the following day.

Implantation of scaffolds. Forty-five male, Sprague-Dawley rats weighing 250-300 grams were purchased from Charles River Laboratories, Inc. (Wilmington, MA) and acclimated to the animal research facility for one week prior to surgery. The rats were fasted overnight prior to surgery to allow their stomachs to decompress. However, water was available during this time.

On the morning of the surgery, the animals were initially placed in an induction chamber with isoflurane (2-3% MAC), and the abdomen was shaved and washed three times with a betadine scrub solution diluted with sterile water.

The animals were then placed on an isoflurane flow-by circuit, titrated to keep respiration normal (average 60bpm) but to provide adequate anesthesia to operative stimuli (1.75-2.0 MAC). All instruments were autoclaved, and a fresh sterile pack was used for each animal. Surgeons operated with standard gowns, hats, and sterile gloves, and all surgeries were performed in a dedicated animal operating room.

A 2cm longitudinal incision was made through the dermis in the midline, and the subcutaneous tissues were bluntly dissected off of the right-sided abdominal musculature to create a subcutaneous pocket. A 1 cm x 1 cm scaffold was placed with the "rough" side facing the fascial surface, and four 4-0 Prolene sutures were used to fix and mark the scaffold location. The midline was closed with five interrupted 4-0 Vicryl sutures.

A dose of buprenorphine (0.02 mg/kg) was administered prior to emergence from anesthesia. Then the animals were placed in a warm, sawdust-free cage until fully alert, moving about the cage and respiring normally. At that point, they were transferred to a standard cage where they were monitored for one hour while they regained normal activity levels. After recovery from the anesthetic, all animals were given food and water ad libitum and returned to central animal housing.

The animals were evaluated for signs of postoperative distress or pain (tachypnea, decreased activity, poor grooming, vocalizing or absent appetite) every 12 hours for the first three days and then daily until the conclusion of the study. If signs of distress or pain were present, buprenorphine was administered to achieve analgesia. The incisions were also observed for signs of infection, and the abdominal wall observed for evidence of seroma, swelling, or local reaction.

Explantation of scaffolds. At seven, twenty-one, and ninety-seven days, the animals were re-anesthetized using the same protocol as the original surgery and placed on a heating pad on the surgical table. The weight of the animal at sacrifice, overall health of the animal, healing of the incision, and presence of induration, seroma, or abscess were noted. A 2cm x 2cm full-thickness section of the abdominal wall including any remaining scaffold material, was removed from each animal and preserved in 10% neutral, buffered formalin. The animals were then humanely euthanized via injected barbiturates while still under anesthesia. A confirmatory bilateral pneumothorax was also created.

Histopathologic analysis. Representative sections of the tissues were embedded in paraffin, cut to a thickness of 3µm, and stained with hematoxylin and eosin (H&E). Each slide was reviewed by a pathologist using an Olympus BX41 microscope and camera (Spot 2, Model# 18.2 Color Mosaic). An initial examination of the entire slide was performed at 20x and 40x original magnification to gather an overview of the tissue reaction. Any changes noted at low magnification, as well as the entire scaffold-host tissue interface, were then examined at 200x and 400x original magnification. A minimum of 10 sites at the scaffold-host interface were selected at 400x original magnification, and the following semi-quantitative scoring system was utilized to characterize the inflammatory response, infiltration of fibroblasts, and neovascularization.

"No reaction": A "0" score for observation of zero inflammatory cells, fibroblasts, or new blood vessels in a high power field at 400x magnification. "Mild": A "1" score for observation of 1-2 inflammatory cells, fibroblasts, or new blood vessels present in a high power field at 400x magnification. "Moderate": A "2" score for observation of inflammatory cells, fibroblasts, or new blood vessels covering half of the high power field at 400x magnification. "Marked": A "3" score for observation of inflammatory cells, fibroblasts, or new blood vessels covering the entire high power field at 400x magnification.

Statistical analyses. Statistical analyses for this study were carried out using GraphPad Prism version 5.0 (GraphPad Software, Inc., San Diego, CA). A two-way analysis of variance was performed to determine whether there were any differences between the semi-quantitative scores due to scaffold type, time of implantation, or an interaction of the two. Significance was set at the 0.05 level.

Results from gross examination. At the time of sacrifice, all of the animals appeared to be healthy. All of the incisions were fully healed with no evidence of induration or abscess. Five possible seromas were observed in three rats in the seven-day group (1 AuNP and 2 SiCNW) and two rats in the twenty-one-day group (1 Decellularized and 1 SiCNW), but all appeared to be superficial and did not interfere with the scaffold-host tissue interface.

Control rats. Hematoxylin and eosin (H&E) stained slides of tissues taken from the control rats revealed normal abdominal wall musculature with no evidence of any tissue reaction at any of the time points evaluated.

Sham rats. After seven days, tissues taken from the sham rats were surrounded by mild to moderate mononuclear chronic inflammatory infiltrate composed of lymphocytes, plasma cells, and histiocytes. An acute inflammatory response characterized by the presence of neutrophils was not detected at this time point. Foreign body reaction to the suture material (with rare multinucleated cells) was also observed. Granulation tissue composed of new small blood vessels and proliferation of fibroblasts was present after seven days, and no fat or muscle necrosis was noted. At both twenty-one and ninety-seven days, no tissue reaction was observed in any of the sham rats.

Decellularized scaffolds. "Decellularized" scaffolds explanted from the rats after seven days were surrounded by a few neutrophils (i.e. acute inflammatory response), and a moderate mononuclear chronic inflammatory infiltrate composed of lymphocytes, plasma cells and histiocytes both at the scaffold-host interface and within the scaffold. No foreign body multinucleated giant cells were identified at this time point. However, marked granulation tissue composed of small blood vessels and fibroblast proliferation was present at the scaffold-host interface. Fat and muscle necrosis were also noted at this time point.

After twenty-one days, the decellularized scaffolds no longer contained any evidence of acute inflammatory infiltrate (i.e. neutrophils), but the moderate chronic mononuclear infiltrate remained as well as mild vascularity and fibroblast infiltration. Again, no foreign body giant cells were observed. Instead of fat and muscle necrosis, regenerated muscle was observed along with replacement of the scaffold by fibroblasts.

After ninety-seven days, only disorganized remnants of the decellularized scaffolds remained, and no evidence of any tissue reaction was observed at this time point. New collagen deposited within the scaffold.

Gold nanoparticle-bionanocomposite scaffolds. "AuNP" bionanocomposite scaffolds explanted after seven days displayed evidence of granulation tissue with edema, as well as marked vascular and fibroblast proliferation replacing the scaffold. The scaffold became disorganized as it was being replaced by new collagen. Acute inflammation, characterized by numerous neutrophils within the scaffold, was observed at this time point. Very mild chronic inflammatory infiltrate (i.e. lymphocytes) was also present at the scaffold-host interface. Mild fat necrosis and moderate interfacial muscle necrosis were observed. At this time point, one slide also showed occasional foreign body multinucleated giant cells associated with the suture material.

After twenty-one days, moderate mononuclear chronic inflammatory infiltrate was present at the interface of the AuNP scaffold with the host tissue as well as within the scaffold. At this time point, evidence of acute inflammation, vascular and fibroblast proliferation and fat and muscle necrosis had disappeared.

After ninety-seven days, very little reaction to the AuNP scaffolds was observed. Only a very focal, mild mononuclear infiltrate remained at the scaffold-host tissue interface. It was difficult to distinguish the original scaffold material from new collagen deposited by the fibroblasts at this time point, and there was also no evidence of scar tissue formation.

Silicon carbide nanowire-bionanocomposite scaffolds. "SiCNW" bionanocomposite scaffolds explanted from the rats after seven days displayed evidence of marked acute inflammation and mild chronic inflammation at the interface of the scaffold with the host tissue as well as infiltrating into the scaffold. However, no foreign body giant cells were observed. Edematous granulation tissue composed of vascular and fibroblast proliferation was present at this time point, along with fat necrosis. Muscle necrosis was also present with vacuolated muscle fibers, inflammatory infiltrate in between the muscle fibers, and regenerated muscle fibers with enlarged nuclei and multinucleation.

After twenty-one days, only marked chronic mononuclear inflammation (composed primarily of lymphocytes) was observed at the interface between the SiCNW scaffold and the host tissue as well as within the scaffold. Acute inflammation was no longer present, and moderate to marked vascularity and fibroblast proliferation with deposition of new collagen were observed. Edema of the surrounding tissue was noted in one rat, and mild muscle necrosis remained at the interface in another rat. No muscle necrosis was observed in the other rats.

After ninety-seven days no reaction was observed in tissues obtained from two of the rats implanted with a SiCNW scaffold. The original scaffold material could not be distinguished from the new collagen deposited by fibroblasts. Tissues from the other two rats displayed evidence of mild to moderate chronic inflammation. No evidence of scar tissue was present in any of the tissues explanted at this time point.

Chronic inflammation. After seven days in vivo the decellularized scaffolds displayed a chronic inflammatory response with a mean score $\pm$ standard error of $2.25 \pm 0.25$, while the AuNP and SiCNW scaffolds both showed only a $1.0 \pm 0.0$ chronic inflammatory response. There was no significant difference in the chronic inflammatory response of the AuNP and SiCNW scaffolds at seven days ($p > 0.05$). However, the inflammatory response to the decellularized scaffolds was significantly higher than either bionanocomposite ($p < 0.001$).

After twenty-one days, the decellularized scaffolds scored $2.0 \pm 0.0$ for chronic inflammatory response, while the AuNP and SiCNW scaffolds scored $1.5 \pm 0.29$ and $3.0 \pm 0.0$ respectively. At this time point, the SiCNW scaffolds incited a significantly greater chronic inflammatory response than either the decellularized or AuNP scaffolds ($p < 0.01$ and $p < 0.001$ respectively). Interestingly, there was no difference in the chronic inflammatory response observed between decellularized and AuNP scaffolds at this time point ($p > 0.05$).

After ninety-seven days there was no evidence of a chronic inflammatory response to the decellularized scaffold (resulting in a score of $0.0 \pm 0.0$). A slight chronic inflammatory response was still observed, however, for both the AuNP and SiCNW scaffolds. These scaffolds scored $0.25 \pm 0.25$ and $0.25 \pm 0.25$ respectively at this time point. Similarly to the results obtained at the twenty-one day time point, there was no significant difference between the reaction observed for decellularized and AuNP scaffolds ($p > 0.05$). A significant difference was observed, however, between the response to AuNP versus SiCNW ($p < 0.01$) and the response between decellularized and SiCNW ($p < 0.001$) with SiCNW scaffolds eliciting the highest inflammatory response.

Fibroblast infiltration and neovascularization. In terms of fibroblast infiltration and neovascularization of the scaffolds, the decellularized scaffolds scored $2.25 \pm 0.25$, while the AuNP and SiCNW scaffolds both scored $3.0 \pm 0.0$ at the seven-day time point. Significantly more fibroblasts infiltrated into the AuNP and SiCNW scaffolds compared to the decellularized scaffolds at this time point, and significantly more new blood vessels were present in the AuNP and SiCNW scaffolds compared to the decellularized scaffolds ($p < 0.05$ for both). However, no difference was observed between the two bionanocomposites (AuNP versus SiCNW, p>0.05) with respect to either fibroblast infiltration or neovascularization.

After twenty-one days the decellularized scaffolds scored 0.25±0.25 for fibroblast infiltration and neovascularization, while AuNP and SiCNW scaffolds scored 0.25±0.25 and 2.5±0.29 respectively. Significantly more fibroblasts and new blood vessels were present in the SiCNW scaffolds compared to the AuNP scaffolds at this time point (p<0.001). Relative to the decellularized scaffolds, significantly fewer fibroblasts and new blood vessels were found in the AuNP scaffolds (p<0.01), and significantly more fibroblasts and new blood vessels were found in the SiCNW scaffolds (p<0.001).

At the ninety-seven day time point, none of the scaffolds showed any evidence of granulation tissue, resulting in scores of 0.0±0.0 for all three scaffolds with respect to fibroblast infiltration and neovascularization. Only disorganized, degraded scaffolds were observed with no host reaction and very mild fibrosis. Statistically, there were no significant differences between the scaffolds at this time point (p>0.05).

The results of the two-way analysis of variance performed on the semi-quantitative scores indicated that the biocompatibility of the scaffolds (i.e. inflammatory response, fibroblast infiltration, and neovascularization) was significantly affected by both the type of scaffold implanted and the length of time the scaffold was implanted. It should be noted that there was also a significant interaction between these two factors. Thus, it was difficult to interpret the results for each factor individually without also considering the interaction of the factors.

At the seven-day time point, the control tissues displayed normal architecture with no adverse tissue reaction, while the sham tissues displayed some degree of chronic inflammation, fibroblast infiltration, and neovascularization. This was the expected result since the rats in the sham category underwent a surgical procedure causing slight tissue injury, while rats in the control category did not. Decellularized scaffolds elicited essentially the same response as the sham operation (p>0.05) at this time point, indicating that the decellularized scaffolds did not cause any additional inflammatory response beyond that expected due to the surgical procedure. Both bionanocomposite scaffolds displayed significantly less inflammation and significantly more fibroblast infiltration and neovascularization than the decellularized scaffolds at the seven-day time point. However, no significant differences were observed between the two different types of nanomaterials at the seven-day time point. These results imply that utilizing nanomaterials as an addition to an ECM scaffold is beneficial for reducing the inflammatory response to these scaffolds and promoting early deposition of granulation tissue. It is likely that the nanomaterials present on the surface of the scaffolds encouraged early cell adhesion and infiltration by influencing the adsorption and confirmation of proteins onto the scaffolds.

It is well known that proteins adsorb onto the surface of an implant almost immediately after implantation, and studies have shown that proteins important for cell adhesion, such as vitronectin, laminin, fibronectin, and collagen all adsorb at higher concentrations on nanomaterials than on conventional materials. (Christenson EM, et al. Nanobiomaterial applications in orthopedics. Journal of Orthopaedic Research 2007;25:11-22.) Properties unique to nanomaterials such as increased surface energy due to increased grains at the surface may promote the adsorption of these proteins and lead to an improvement in cell adhesion. These properties may also lead to greater influence over subsequent cellular signaling cascades, differentiation, and gene expression. (Balasundaram G, Webster TJ. A perspective on nanophase materials for orthopedic implant applications. Journal of Materials Chemistry 2006;16:3737-3745; Dillow AK, Lowman AM. Biomimetic Materials and Design. New York, NY: Marcel Dekker, Inc.; 2002. 29-53 p.; Kay S, Thapa A, Haberstroh KM, Webster TJ. Nanostructured polymer/nanophase ceramic composites enhance osteoblast and chondrocyte adhesion. Tissue Engineering 2002;8:753-761). Many cell types such as osteoblasts, fibroblasts, and endothelial cells are considered "anchorage-dependent." The initial adsorption of proteins on the surface of the implant is extremely influential over their adhesion to the surface and ultimately, the successful integration of the implant into the host tissue. (Webster TJ, Ergun C, Doremus RH, Siegel RW, Bizios R. Specific proteins mediate enhanced osteoblast adhesion on nanophase ceramics. Journal of Biomedical Materials Research 2000;51:475-483).

In addition to protein adsorption, the conformation of the adsorbed proteins is also influential over cell adhesion. When these proteins adopt a more unfolded conformation, more cell-adhesive sites (such as the well-known arginine-glycine-aspartic acid (RGD) amino acid sequence) are exposed, increasing the potential for more cells to bind to the substrate through their membrane receptors. Webster et al. have demonstrated that the protein vitronectin adopts a more unfolded conformation on nanomaterials compared to conventional materials. (Webster TJ, Schadler LS, Siegel RW, Bizios R. Mechanisms of enhanced osteoblast adhesion on nanophase alumina involve vitronectin. Tissue Engineering 2001;7:291-301). They utilized surface-enhanced Raman scattering (SERS) to show that a larger number of hydrogen bonds were formed between the nanomaterials and the phenol groups present in vitronectin compared to conventional materials. These results demonstrated that vitronectin unfolded to a greater extent, allowing it to form more bonds with the nanomaterial surface. (Webster TJ, Schadler LS, Siegel RW, Bizios R. Mechanisms of enhanced osteoblast adhesion on nanophase alumina involve vitronectin. Tissue Engineering 2001;7:291-301). Thus, it is possible that during this study proteins adsorbed and unfolded to a greater extent on the bionanocomposites and influenced the cellular response to these scaffolds.

It is also interesting to note that as early as seven days after implantation, both bionanocomposite scaffolds (AuNP and SiCNW) displayed infiltration of inflammatory cells into the scaffolds and evidence of scaffold remodeling. These are important observations because chemical crosslinkers were utilized to attach the nanomaterials to the ECM.

Studies have shown that cellular infiltration into a crosslinked scaffold and tissue remodeling may be slowed by excessive crosslinking (Abraham GA, Murray J, Billiar K, Sullivan SJ. Evaluation of the porcine intestinal collagen layer as a biomaterial. Journal of Biomedical Materials Research 2000;51:442-452) or the release of cytotoxic residues. (Chang Y, Tsai CC, Liang HC, Sung HW. In vivo evaluation of cellular and acellular bovine pericardia fixed with a naturally occurring crosslinking agent (genipin). Biomaterials 2002;23:2447-245). The chemical crosslinkers utilized during this study were 1-ethyl-3-[3-dimethylaminopropyl]carbodiimide (EDC) and N-Hydroxysuccinimide (NHS). These crosslinkers are considered "zero-length" crosslinkers, meaning that they do not become part of the covalent bond. Their purpose is solely to activate the carboxyl groups on the ECM and drive the formation of an amide bond between the ECM and the nanomaterials. (Khor E. Methods for the treatment of collagenous tissues for bioprostheses. Biomaterials 1997;18:95-105). For this reason, there is no threat of a cytotoxic response to these crosslinkers because they do not remain within the bionanocomposite scaffolds and cannot leach out as the scaffolds degrade. In addition, low concentrations of EDC and NHS (2mM and 5mM respectively) were utilized during this study to prevent excessive crosslinking. Studies have shown that higher concentrations of EDC (100mM) can lead to excessive crosslinking that slows cellular infiltration and tissue remodeling. (Abraham GA, Murray J, Billiar K, Sullivan SJ. Evaluation of the porcine intestinal collagen layer as a biomaterial. Journal of Biomedical Materials Research 2000;51:442-452). Thus, the results obtained from this study confirmed that excessive crosslinking of the ECM did not occur since both bionanocomposite scaffolds were beginning to be remodeled as early as seven days after implantation and were completely remodeled by ninety-seven days. These results imply that the role of the crosslinkers utilized in this study was limited to simply attaching the nanomaterials to the ECM rather than crosslinking the collagen molecules of the ECM.

After twenty-one days, there was no difference ($p > 0.05$) between tissues taken from the control rats and tissues taken from rats that underwent the sham operation. These results indicate that there were no long-term effects of the surgical procedure and that any tissue injury resulting from the surgery itself was completely healed without the formation of scar tissue by twenty-one days. At this time point, the decellularized scaffolds were beginning to be replaced by healthy tissue and had elicited significantly more chronic inflammation, fibroblast infiltration, and neovascularization compared to the sham operation ($p < 0.001$). A difference was also observed between the two bionanocomposites at twenty-one days. The SiCNW scaffolds elicited significantly more chronic inflammation, fibroblast infiltration, and neovascularization than either the AuNP scaffolds ($p < 0.001$) or the decellularized scaffolds ($p < 0.01$). The AuNP scaffolds appeared to be almost completely remodeled at this time point with a disappearance of most granulation tissue. Decellularized scaffolds still contained granulation tissue with significantly more fibroblasts and new blood vessels than AuNP scaffolds ($p < 0.01$) indicating that the healing process was still progressing.

By ninety-seven days there was no difference in the chronic inflammatory response, fibroblast infiltration, or neovascularization observed in tissues taken from the control, sham, decellularized, or AuNP groups. All three types of scaffolds (decellularized, AuNP, and SiCNW) were degraded with no evidence of any adverse tissue reactions or scar formation. Healthy, new collagen had also been deposited by fibroblasts by this time point. The only significant result after ninety-seven days was a slight, chronic inflammatory response to the SiCNW scaffolds, but this response was very mild and only observed in two of the rats in this group.

In general, some key differences were observed between the performances of the AuNP and SiCNW bionanocomposite scaffolds in vivo. Most notably, the scaffold remodeling process appeared to progress more rapidly in tissues exposed to AuNP scaffolds compared to tissues exposed to either SiCNW or decellularized scaffolds. In addition, the cellular response to the SiCNW scaffolds and remodeling process was more aggressive and persisted longer than that observed for either the AuNP or decellularized scaffolds. It is possible that the properties of nanomaterials in general such as the improved protein adsorption and unfolding discussed earlier played a role in the overall differences between decellularized scaffolds and bionanocomposite scaffolds. In addition to these general properties, it is also possible that a unique property of gold nanoparticles such as the ability to scavenge free radicals (Hsu SH, Tang CM, Tseng HJ. Biocompatibility of poly(ether)urethane-gold nanocomposites. Journal of Biomedical Materials Research Part A 2006;79:759-770) played a role in accelerating the healing of tissues exposed to AuNP scaffolds.

There were no long-term, adverse reactions to either bionanocomposite, and after ninety-seven days there were no differences between tissues exposed to AuNP versus SiCNW scaffolds. Both had been remodeled normally, with mild fibrosis and no scar tissue formation.

Example 3: AuNP-crosslinked bionanocomposite, Surgisis, and Permacol Study

Experimental design. The following four biologic tissue scaffold materials were implanted into the abdominal walls of fifteen female, Landrace pigs. The abdominal wall of each pig was divided into four regions separated from each other by at least one inch on each side. A 16cm$^2$ piece of each of the four types of scaffolds was placed into these quadrants, and the placement location of each type of scaffold was randomly determined for each pig. Five pigs were sacrificed at each of the three time points (one, three, and six months). Full thickness sections of the abdominal walls and any remaining scaffold materials were recovered at these times and subjected to histological analysis.

"Non-crosslinked" (Surgisis) scaffolds: This scaffold material was comprised of several layers of non-crosslinked porcine small intestine submucosa. (Cook Biotech Incorporated, West Lafayette, IN) "Slightly crosslinked" (AuNP-crosslinked) scaffolds: This scaffold material was comprised of one layer of porcine diaphragm tissue that was crosslinked with mercaptoethylamine (MEA)-functionalized gold nanoparticles (AuNP) in combination with EDC and NHS. "Moderately crosslinked" (EDC-crosslinked) scaffolds: This scaffold material was comprised of one layer of porcine diaphragm tissue that was crosslinked twice using the chemical crosslinkers 1-ethyl-3-[3-dimethylaminopropyl]carbodiimide (EDC) and N-Hydroxysuccinimide (NHS). "Heavily crosslinked" (Permacol) scaffolds: This scaffold material was comprised of one layer of hexamethylene diisocyanate crosslinked porcine dermis. (Tissue Science Laboratories Incorporated, Andover, MA)

Preparation of scaffolds. Porcine diaphragms were harvested and decellularized as described in example 2 and example 1, respectively. The crosslinking solution was comprised of a 50:50(v/v) solution of acetone and 1x phosphate buffered saline (PBS) (pH=7.4) with a final concentration of 2mM EDC and 5mM NHS. The NHS was initially dissolved in a small volume of dimethylformamide (DMF), and the EDC was likewise dissolved in a small volume of 0.1M 2-(N-Morpholino)ethanesulfonic acid (MES) with 0.5M sodium chloride (NaCl) (pH 6.0). The two solutions were immediately mixed together and added to the acetone/PBS solution. The tissues were then reacted with this solution at ambient temperature for 15 minutes to activate the carboxyl groups present on the collagen molecules. Meanwhile, the gold nanoparticles (AuNP) were functionalized with $\beta$-mercaptoethylamine hydrochloride (MEA) at a concentration of 15$\mu$M MEA in order to functionalize the AuNP with terminal amine groups to promote covalent bonding to the porcine diaphragm tendon. After this incubation period, 3.0mL of MEA-functionalized AuNP were pipetted on top of tissues requiring crosslinking with AuNP. The EDC-crosslinked tissue group did not receive an addition of AuNP but rather, remained in the crosslinking solution for 24 hours. Regardless of the treatment, both EDC-crosslinked and AuNP-crosslinked scaffolds were incubated at ambient temperature for 24 hours. The "EDC-crosslinked" scaffolds were then incubated in fresh crosslinking solution for an additional 24 hours. During this time, the AuNP-crosslinked scaffolds were rinsed with 1x PBS. Subsequently, both types of scaffolds were subjected to 48 hours of rinses with PBS in which the PBS was exchanged after 24 hours.

The two commercially-available products (Surgisis and Permacol) were received in a sterile package, and thus were not subjected to any further sterilization. The EDC-crosslinked and AuNP-crosslinked scaffolds, however, were sterilized by an aqueous solution of 0.1 %(v/v) peracetic acid and 1.0M NaCl at room temperature for 24 hours with constant agitation. This treatment was followed by 48 hours of rinses with sterile 1x PBS in which the PBS was changed after 24 hours. The tissues were then stored in 70%(v/v) ethyl alcohol at 4°C until they were surgically implanted.

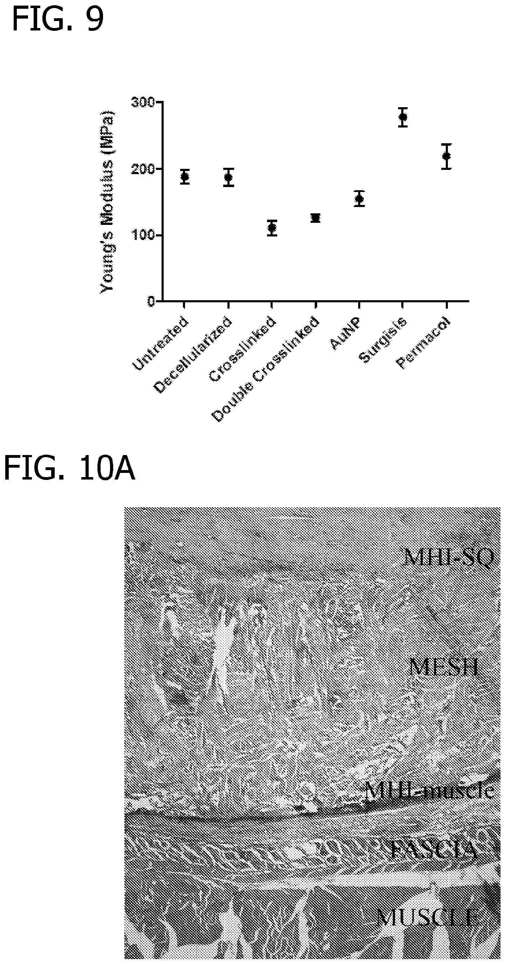

Uniaxial Testing. During the tensile testing of the four materials, the Young's modulus for each material was measured as well. The graph of the Young's modulus for the various scaffolds is shown in Figure 9. The Tukey's statistical test results are detailed in the table below.

| Tukey's Multiple Comparison Test | Significant? P < 0.05? | Summary |
|---|---|---|
| Untreated vs Decellularized | No | ns |
| Untreated vs Crosslinked | Yes | ** |
| Untreated vs Double Crosslinked | Yes | * |
| Untreated vs AuNP | No | ns |
| Untreated vs Surgisis | Yes | *** |
| Untreated vs Permacol | No | ns |
| Decellularized vs Crosslinked | Yes | ** |
| Decellularized vs Double Crosslinked | Yes | * |
| Decellularized vs AuNP | No | ns |
| Decellularized vs Surgisis | Yes | *** |
| Decellularized vs Permacol | No | ns |
| Crosslinked vs Double Crosslinked | No | ns |
| Crosslinked vs AuNP | No | ns |
| Crosslinked vs Surgisis | Yes | *** |
| Crosslinked vs Permacol | Yes | *** |
| Double Crosslinked vs AuNP | No | ns |
| Double Crosslinked vs Surgisis | Yes | *** |
| Double Crosslinked vs Permacol | Yes | *** |
| AuNP vs Surgisis | Yes | *** |
| AuNP vs Permacol | Yes | * |

(continued)

| Tukey's Multiple Comparison Test | Significant? P < 0.05? | Summary |
|---|---|---|
| Surgisis vs Permacol | Yes | * |

Implantation of scaffolds. Fifteen female, Landrace pigs weighing 60-80 pounds were purchased from the Sinclair Research Farm Swine Complex of the University of Missouri (Columbia, Missouri) and acclimated to the animal research facility for one week prior to surgery. To prevent behavioral distress due to the use of abdominal binders post-operatively, the pigs were trained to accept abdominal bandaging during this one-week acclimation period.

The animals were fasted overnight prior to surgery, and anesthetic induction agents (Xylazine and Telazol) were administered in animal housing on the morning of surgery, prior to transport to the operating room where they were placed on a heating pad. After intubation and administration of Isoflurane, the pigs were shaved from sternum to crotch approximately 10-12 inches from the midline bilaterally and washed with a betadine scrub solution to remove stray hair. An isotonic sodium chloride solution was infused via an intravenous line in one of the superficial ear veins throughout the operation.

Following full sterile preparation of the animal, a midline skin incision was created using a number 15 scalpel. The skin and subcutaneous tissue were dissected off the anterior body wall musculature using electrocautery. The dissection extended at least 12cm down each side laterally and at least 22cm vertically. After the anterior abdominal wall was exposed, 4cm x 4cm pieces of the four types of scaffolds were placed in each pig with at least 2cm between each scaffold. To mark the starting location of each scaffold, 2-0 Prolene sutures were placed in the fascia along each of the four sides of the material. The subcutaneous tissues were re-approximated using 0 Vicryl running sutures and the skin closed with skin staples. Triple antibiotic ointment and dressings were applied to the wound, and a standard abdominal binder was placed over the abdomen to reduce post-operative seromas.

Shortly after cessation of Isoflurane anesthesia, the first dose of post-operative Buprenex was administered intravenously. The second dose of post-operative Buprenex was given at the same dosage intramuscularly approximately 6-12 hours after the first. Buprenex was then administered every 6-12 hours at full dosage for 24-72 hours post-operatively based on the pain needs of each individual pig.

Monitoring for recovery was performed every 15 minutes until the pigs were fully awake and on their feet. Monitoring included heart rate, breathing, and pain response. The animals were placed in a large animal transport carrier at the end of surgery to prevent potential thrashing and injury as they emerged from anesthesia. They were kept in confinement, in a quiet area, until they were standing and calm.

The binders were removed one week post-operatively to allow wound monitoring, and the staples were removed after 2 weeks. There was free access to food and water throughout the course of this study, but the calories were limited to approximately two-thirds of a standard diet to prevent the animals from growing to an unmanageable size by the end of the six-month experiment.

Explantation of scaffolds. The pigs were observed for one, three, or six months with five pigs in each group. At the time of sacrifice, the pigs were re-anesthetized using the same protocol as the original surgery and placed on heating pads on the surgical table. A midline incision identical to the original incision was created, and the original dissection re-exposed. Full-thickness sections of the abdominal wall, including all four scaffold sites and 1cm of surrounding tissue, were harvested from each animal and preserved in 10% neutral, buffered formalin. Once all specimens were collected, the pigs were humanely euthanized via injected barbiturates while still under anesthesia. A confirmatory bilateral pneumothorax was also created.

Histopathology. A 3.0cm section of each explanted scaffold (also referred to as biologic "mesh") was placed in a block. This section contained a 0.5cm region of normal tissue that was separated by suture at the scaffold margin from a 2.5cm region of scaffold. The samples were then embedded in paraffin, cut to a thickness of 5$\mu$m, and stained with hematoxylin and eosin (H&E). The mesh-host interface (MHI) was evaluated by a pathologist along the entire 2.5cm length from the scaffold margin to the center using a Zeiss Axiophot microscope, and images were acquired using an Olympus DP70 digital camera.

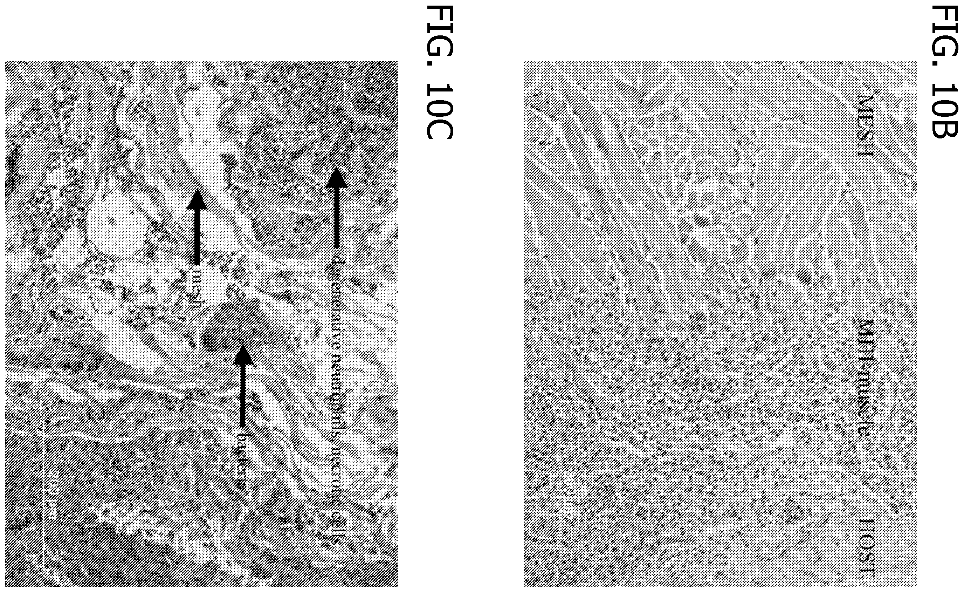

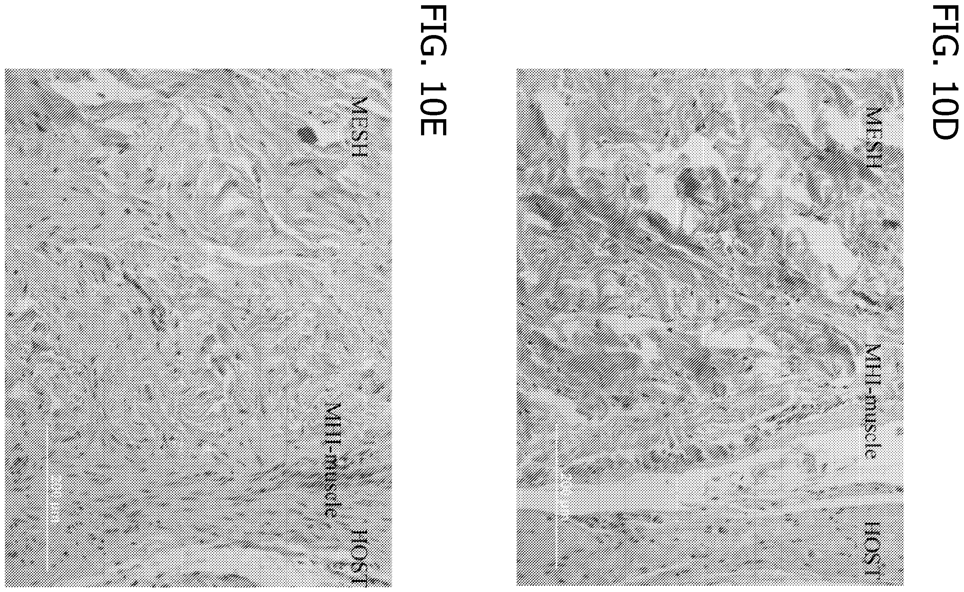

Tissue evaluation. Each slide consisted of abdominal wall musculature with the overlaying fascia. The scaffolds overlaid the fascia, and this interface was designated "MHI-muscle side." Many sections contained host subcutaneous tissue overlaying the scaffold, and this interface was designated "MHI-SQ side." However, in many sections, this overlaying tissue was either not harvested or not apparent on the slide. Figure 10 depicts a photograph of a representative slide showing all of these layers. The cellularity, presence of multinucleated giant cells, and neovascularization were scored according to a semi-quantitative scale (Table 3.1) found in the literature (Valentin JE, Badylak JS, McCabe GP, Badylak SF. Extracellular matrix bioscaffolds for orthopaedic applications. A comparative histologic study. Journal of Bone and Joint Surgery American 2006;88:2673-2686) at ten sites approximately 2mm apart along the MHI-muscle side from the periphery (scaffold margin) to the center of the scaffold. A description of the reaction at the scaffold margin, the MHI-muscle side, and the MHI-SQ side was also recorded for each slide.

No scores were calculated at the one month time point for one of the Surgisis scaffolds due to separation of the scaffold from the host or for two of the EDC-crosslinked scaffolds due to the inability to identify the MHI.

Table 3.1

| Score | 0 | 1 | 2 | 3 |
|---|---|---|---|---|
| Cellularity | 0-50 cells per 400x field | 51-100 cells per 400x field | 101-150 cells per 400x field | >150 cells per 400x field |
| Multinucleated Giant Cell Presence | 0 MNGCs per 400x field | 1-2 MNGCs per 400x field | 3-4 MNGCs per 400x field | >5 MNGCs per 400x field |
| Neovascularization | 0-1 blood vessel per 400x field | 2-5 blood vessels per 400x field | 6-10 blood vessels per 400x field | >10 blood vessels per 400x field |

Table 3.1 Semi-quantitative scoring system

Statistical analyses. Statistical analyses for this study were carried out using GraphPad Prism version 5.0 (GraphPad Software, Inc., San Diego, CA). A one-way analysis of variance was performed, followed by a Tukey's post-test to determine whether differences existed between the mean scores for cellularity, presence of multinucleated giant cells, and neovascularization for the four scaffolds at the one month time point. Significance was set at the 0.05 level. A two-way analysis of variance will be performed once the three month and six month data are collected to determine whether there are any differences in these scores due to the type of scaffold, time of implantation, or an interaction of these factors.

Semi-quantitative scoring results (one month). The mean scores for cellularity, presence of multinucleated giant cells, and neovascularization after one month in vivo are reported below $\pm$ standard error of the mean. Surgisis scored $0.25 \pm 0.25$ for cellularity, while the score for the slightly crosslinked (AuNP-crosslinked) scaffold was significantly lower at $0.08 \pm 0.08$ ($p < 0.05$). Interestingly, there was no difference between the AuNP-crosslinked scaffold and that of the moderately crosslinked (EDC-crosslinked) scaffold which had a score of $0.23 \pm 0.15$ for cellularity ($p > 0.05$). The most dramatic cellularity score was that recorded for the heavily crosslinked Permacol scaffold which had a score of $2.3 \pm 0.42$. This score was significantly higher than that of the moderately EDC-crosslinked scaffold ($p < 0.01$) and the slightly AuNP-crosslinked scaffold ($p < 0.001$), but there was no difference between Permacol and Surgisis in terms of cellularity ($p > 0.05$).

None of the scaffolds evaluated displayed a marked presence of multinucleated giant cells at the one month time point. The Surgisis scaffold scored $0.05 \pm 0.05$ for presence of multinucleated giant cells. The slightly AuNP-crosslinked scaffold scored $0.16 \pm 0.14$, but this did not represent a significantly greater number of multinucleated giant cells relative to the non-crosslinked Surgisis scaffold ($p > 0.05$). The moderately EDC-crosslinked scaffolds scored $0.10 \pm 0.06$ for presence of multinucleated giant cells, and again, there was no difference between the slightly crosslinked and moderately crosslinked scaffolds ($p > 0.05$). Similarly, the heavily crosslinked Permacol scaffolds scored $0.14 \pm 0.12$, and there was no difference between the presence of multinucleated giant cells for moderately crosslinked and heavily crosslinked scaffolds ($p > 0.05$).

Similar to the results for presence of multinucleated giant cells, all of the scaffolds evaluated during this study scored equally in the category of neovascularization at the one month time point. Surgisis scored $0.83 \pm 0.11$, while the slightly AuNP-crosslinked scaffold scored $0.82 \pm 0.12$ (Surgisis vs. AuNP, $p > 0.05$). There was also no significant difference between the slightly AuNP-crosslinked scaffolds and the moderately EDC-crosslinked scaffolds ($p > 0.05$). Similarly, there was no difference ($p > 0.05$) between the neovascularization scores for the moderately EDC-crosslinked scaffolds ($0.53 \pm 0.18$) and the heavily crosslinked Permacol scaffolds ($0.60 \pm 0.13$).

One month pig data on vascularity.

| Animal ID | Vascularity |
|---|---|
| | Permacol |
| 6.4 | 0.3 |
| 7.2 | 1 |
| 8.1 | 0.7 |
| 9.4 | 0.3 |
| 10.3 | 0.7 |

(continued)

| Animal ID | Vascularity |
|---|---|
| **Average** | 0.6 |
| Surgisis | |
| 6.2 | n/a |
| 7.1 | 0.5 |
| 8.4 | 0.9 |
| 9.2 | 1 |
| 10.1 | 0.9 |
| **Average** | 0.825 |
| Diaphragm-AuNP | |
| 6.1 | 0.8 |
| 7.4 | 1.1 |
| 8.2 | 0.4 |
| 9.3 | 1 |
| 10.4 | 0.8 |
| **Average** | 0.82 |
| Diaphragm | |
| 6.3 | n/a |
| 7.3 | n/a |
| 8.3 | 0.6 |
| 9.1 | 0.8 |
| 10.2 | 0.2 |
| **Average** | 0.533333333 |

Three month pig data on Vascularity and Connective Tissue organization

| Animal ID | Vascularity-periphery | Vascularity-center | Connective tissue organization |
|---|---|---|---|
| Permacol | | | |
| 267-1 | 0.8 | 0.4 | 0 |
| 268-2 | 0.9 | 0.6 | 0 |
| 269-4 | 1.8 | 0.3 | 0 |
| 270-1 | 2 | 1 | 0 |
| 271-3 | 1.3 | 1 | 0 |
| **Average** | 1.36 | 0.66 | 0 |
| Surgisis | | | |
| 267-2 | 1.4 | 1.5 | 1 |
| 268-1 | 1.8 | 2.6 | 2 |
| 269-2 | 1.4 | 0.2 | 1 |
| 270-2 | 1.9 | 1.9 | 2 |
| 271-2 | 1.4 | 1 | 2 |
| **Average** | 1.58 | 1.44 | 1.6 |
| Diaphragm | | | |
| 267-3 | 2.3 | 2.2 | 2 |
| 268-3 | 2.7 | 2.1 | 2 |
| 269-1 | 1.9 | 1.9 | 2 |
| 270-3 | 1.9 | 1.5 | 2 |
| 271-4 | 1.4 | 1.1 | 2 |
| **Average** | 2.04 | 1.76 | 2 |
| AuNP-Diaphragm Nanocomposite | | | |

(continued)

| Animal ID | Vascularity-periphery | Vascularity-center | Connective tissue organization |
|---|---|---|---|
| 267-4 | 2 | 1.8 | 3 |
| 268-4 | 2.6 | 2.4 | 2 |
| 269-3 | 1.9 | 1.7 | 2 |
| 270-4 | 1.2 | 1.3 | 1 |
| 271-1 | 2.6 | 2.5 | 2 |
| **Average** | 2.06 | 1.94 | 2 |

In summary, after 3 months, the diaphragm mesh scored high in connective tissue organization; better than either Permacol or Surgisis. The diaphragm with AuNPs mesh scored the highest in vascularity, both periphery and center.

Six month pig data on Vascularity and Connective Tissue organization

| Animal ID | Vascularity-periphery | Vascularity-center | Connective tissue organization |
|---|---|---|---|
| | | Permacol | |
| 169-4 | 1 | 0.4 | 0 |
| 170-2 | 1.7 | 0.7 | 0 |
| 171-3 | 0.9 | 0.1 | 0 |
| 172-2 | 0.9 | 0.4 | 0 |
| 173-3 | 0.8 | 0.4 | 0 |
| **Average** | 1.06 | 0.4 | 0 |
| | | Surgisis | |
| 169-1 | 1.7 | 1.8 | 2 |
| 170-1 | 1.6 | 1.5 | 2 |
| 171-2 | 1.8 | 2 | 2 |
| 172-1 | 2.1 | 2 | 2 |
| 173-2 | 1.8 | 1.3 | 2 |
| **Average** | 1.8 | 1.72 | 2 |
| | | Diaphragm | |
| 169-3 | 1.3 | 1.8 | 3 |
| 170-3 | 1.6 | 1.1 | 2 |
| 171-1 | 1 | 1.5 | 1 |
| 172-4 | 2.6 | 1.8 | 2 |
| 173-1 | 1.6 | 1.4 | 2 |
| **Average** | 1.62 | 1.52 | 2 |
| | | AuNP-Diaphragm nanocomposite | |
| 169-2 | 1.9 | 1.9 | 2 |
| 170-4 | 1.4 | 2 | 2 |
| 171-4 | 1.3 | 2.5 | 2 |
| 172-3 | 1.1 | 1.2 | 2 |
| 173-4 | 1.1 | 0.5 | 2 |
| **Average** | 1.36 | 1.62 | 2 |

In summary, after 6 months, the diaphragm mesh scored high in connective tissue organization; better than Permacol. Surgisis also scored high, but the tissue integrity was compromised in that it had started to degrade and delaminate. Thus its data is skewed. The diaphragm with AuNPs mesh scored higher in center vascularity than Permacol or just diaphragm. Typically, biologic mesh should incorporate and deposit new collagen. We should see a decrease in overall vascularity as compared to 3 months. Indeed this is the case with all of the implanted meshes, except Surgisis.

At the six month point, there was a significant difference between the AuNP-diaphragm nanocomposite and Permacol between periphery vascularity and center vascularity. In fact, the AuNP-diaphragm nanocomposite had the only negative average; meaning more vessels were found in the center of the mesh than at the periphery.

Summary of histopathology (one month). After one month, the non-crosslinked scaffolds (Surgisis) were markedly disorganized, and there was an abundant fibrous tissue reaction surrounding the scaffolds. The layers of these scaffolds were infiltrated and separated by fibrous tissue, inflammatory cells, and blood vessels. Colonies of gram positive coccoid bacteria were also observed in three out of five of the Surgisis scaffolds at the one month time point.

The slightly AuNP-crosslinked scaffolds remained mostly intact after one month in vivo and were infiltrated by blood vessels, many scattered fibroblasts, and very few inflammatory cells. A mild fibrous tissue reaction with a few inflammatory cells and multinucleated giant cells was also observed at the MHI-muscle side.

The moderately EDC-crosslinked scaffolds produced the most variable results after one month with variable fibrous tissue reaction surrounding these scaffolds. In a few animals, the scaffolds appeared disorganized and infiltrated by abundant fibrous tissue. In other animals, the inflammatory reaction was minimal, and there was some infiltration of blood vessels and fibroblasts.

The heavily crosslinked Permacol scaffolds remained fully intact after one month in vivo, and the borders of the scaffolds were clearly demarcated. These scaffolds were infiltrated (mostly on the periphery at the MHI) with marked inflammatory cells and scattered blood vessels and fibroblasts. There was also an abundant fibrous tissue reaction surrounding the scaffolds.

After one month in vivo, the Permacol scaffolds scored the highest of all four biologic scaffold materials in the category of "cellularity" according to the semi-quantitative scoring system. It should be noted that the total number of cells found in a high powered field (400x) were counted without discrimination between cell types. The resulting score, therefore, does not provide any information about the types of cells found in each scaffold (i.e. neutrophils, mononuclear cells, multinucleated giant cells, etc.) This score also does not take into account the number of cells at the periphery of the scaffold versus the number infiltrating into the scaffold. This information was recorded in the form of qualitative observations as the slides were evaluated. Taken by itself, the semi-quantitative cellularity score for the Permacol scaffolds after one month in vivo was surprising given literature documenting slow or non-existent cellular infiltration into heavily-crosslinked scaffolds such as Permacol. However, qualitative descriptions of each slide revealed that the majority of these cells were found at the periphery of the scaffolds with little cellular infiltration into the scaffold itself. These qualitative descriptions were more consistent with what was expected for a heavily-crosslinked material such as the Permacol scaffold. The cells at the periphery were predominately mononuclear cells, but a moderate number of neutrophils were also observed, along with a few multinucleated giant cells. It should also be noted, that the borders of the Permacol scaffolds were clearly demarcated indicating very little scaffold disorganization or degradation, which was also consistent with what was expected for a heavily-crosslinked material such as the Permacol scaffold. Moderate fibrous tissue was also observed at the mesh margin, which could indicate the beginning of a fibrous encapsulation of the Permacol scaffolds.

The other commercially-available product evaluated during this study was Surgisis, a non-crosslinked, layered porcine small intestine submucosa scaffold. This scaffold also scored fairly high with regard to cellularity. It should be noted that there was no significant difference (p>0.05) between the cellularity scores for Surgisis versus Permacol scaffolds. This was a surprising result since these two scaffold materials represent opposite ends of the crosslinking spectrum. Surgisis is representative of several non-crosslinked scaffold materials that have been shown to allow rapid cellular infiltration and scaffold remodeling, while Permacol represents heavily-crosslinked scaffolds that have been shown to slow cellular infiltration and remodeling. However, there were some major differences in the qualitative data for these scaffolds. After just one month in vivo, the Surgisis scaffolds were already markedly disorganized and, in some cases, the scaffold material had even "balled up." In addition, cells were observed infiltrating into the Surgisis scaffolds, which contrasts with the Permacol scaffolds in which the majority of the cells were observed at the periphery. In general, the layers of the Surgisis scaffolds were shown to be separated by fibrous tissue, mononuclear cells, neutrophils, multinucleated giant cells, blood vessels, and in three out of five pigs, colonies of gram positive coccoid bacteria. In one of the Surgisis scaffolds, the layers were separated by necrotic cells (neutrophils and nuclear/cellular debris) and bacteria. Studies have shown that porcine ECM-based scaffolds possess antibacterial properties, so it is unclear why so many of the Surgisis scaffolds possessed colonies of bacteria at the one month time point. It will be interesting to note whether this trend continues at later time points.

The two novel scaffolds evaluated during this study represented low and moderate levels of carbodiimide crosslinking. It was hypothesized that the role of the carbodiimide crosslinkers utilized in the preparation of the slightly-crosslinked AuNP scaffolds would be primarily to drive the formation of an amide bond between the amine groups on the AuNP and the carboxyl groups in the porcine tissue rather than to excessively crosslink the collagen molecules to each other. On the other hand, the EDC-crosslinked scaffolds were crosslinked twice in an effort to produce a more moderately crosslinked scaffold in which the collagen molecules were bound to each other. It was hypothesized that the gold nanoparticles would also affect cellular behavior by influencing protein adsorption and conformation. Thus, the AuNP scaffolds represented an ideal combination of slight crosslinking to improve mechanical properties and achieve adequate, but not excessive resistance to enzymatic degradation. The addition of nanomaterials represented a novel way to further influence cellular response to the scaffolds.

With regard to the scores for cellularity after one month in vivo, there was no significant difference between the AuNP-crosslinked scaffolds and the EDC-crosslinked scaffolds (p>0.05). Going back to the qualitative descriptions, it was found that the AuNP-crosslinked scaffolds contained mostly blood vessels, scattered fibroblasts, and only a few mononuclear cells inside the scaffolds at the one-month time point. These results are indicative of granulation tissue and the initial stages of remodeling. Data from later time points is needed confirm that these findings represent early granulation tissue formation and scaffold remodeling. The three and six month data will also help to elucidate differences between the scaffolds that may be due to the addition of gold nanoparticles. It is difficult to speculate whether the gold nanoparticles are responsible for the positive characteristics observed after only one month, but it is certainly possible that the gold nanoparticles played a role in improving early protein adsorption and unfolding. It should also be noted that mild fibrous tissue was observed along the periphery of the AuNP scaffolds, along with a few mononuclear cells, neutrophils, and multinucleated giant cells. The hope is that this mild reaction represents the beginning of scaffold remodeling rather than fibrous encapsulation. Again, data from the three and six month time points will help make this determination.

The EDC-crosslinked scaffolds yielded the most variable results of all of the scaffolds investigated during this study. These scaffolds displayed a marked fibrous tissue reaction with inflammatory cells and multinucleated giant cells at the outermost edge of the scaffold (likely due to suture reaction) with moderate fibrous tissue extending along the scaffold-host interface. Again, it is unknown at this early time point whether this fibrous tissue will eventually become a fibrous encapsulation of the scaffold. At the scaffold-host interface, a moderate number of multinucleated giant cells, mononuclear cells, and blood vessels were also observed, and in many of the EDC-crosslinked scaffolds, blood vessels, scattered fibroblasts, and mild fibrous tissue were observed infiltrating into the center of the scaffolds. These results are similar to the AuNP scaffolds in that cells, blood vessels, and fibroblasts were able to infiltrate the scaffolds. These observations confirm that a moderate, rather than excessive, level of crosslinking was achieved by the double EDC treatment.

At this early time point (i.e. one month), there were no differences between any of the four scaffolds with regard to either multinucleated giant cell presence or neovascularization of the scaffold. It is encouraging that very few multinucleated giant cells were observed in any of the scaffolds, particularly the novel AuNP-crosslinked scaffolds, as this indicates a very mild foreign body reaction. It is well known that a persistent foreign body such as a permanent scaffold can lead to chronic inflammation, foreign body reaction, and ultimately, encapsulation. It will be interesting to observe whether the fibrous tissue and multinucleated giant cells surrounding the scaffolds decrease at later time points as the scaffolds are remodeled or increase due to the formation of a fibrous capsule around the scaffolds.

Overall, the scaffolds displayed the expected behavior with the heavily-crosslinked Permacol scaffolds demonstrating the least disorganization and cellular infiltration and the non-crosslinked Surgisis scaffolds demonstrating the greatest disorganization and cellular infiltration as early as one month after implantation.

Example 4: Electrospun polycaprolactone nanoparticles

The Electrospinning Apparatus. The basic requirements for electrospinning include a suitable solvent to dissolve the polymer, an appropriate solution viscosity and surface tension, an adequate voltage power supply, and an appropriate electrode separation distance between the dispensing needle and ground plate. While all of these parameters are interdependent, the construction of an electrospinning apparatus was the first priority in this project. Once an integrated apparatus was built, it allowed for the easy manipulation of all of the necessary electrospinning parameters.

The electrospinning apparatus was based on a previous design constructed by Sautter et al. from the University of Illinois at Chicago. The design included a semi-isolated system where the polymer solution, syringe pump, syringe, dispensing needle, ground plate were enclosed in a Plexiglas safety box. External components included a high voltage power supply connected to the dispensing needle and ground plate via electrodes, a AC to DC transformer, and an external CPU interface.

The electrospinning apparatus for this project incorporated many of the ideas from the Sautter et al prototype, but certain modifications were implemented in order to make the apparatus more suitable for experimentation. A Spellman 230-30R Series Bench-top High Voltage Power Supply (Valhalla, NY) with a maximum voltage output of 30 kV and a 500$\mu$A current was used to generate a charge build-up on the polymer solution and create an electric field between the dispensing needle and ground plate. The voltage supply was connected to a (18-22) gauge I&J Fisnar (Fairlawn, NJ), 1.5" blunt-end stainless steel dispensing needle and a 6"x6", 3/32" thick, copper McMaster-Carr (Atlanta, GA) ground plate. Corning, 1"x3" microscope slides were place on the copper ground plate in order to collect electrospun samples for analysis.

A McMaster-Carr ceramic rod 5/8" diameter and 12" long isolated the high voltage needle from the rest of the system. The ceramic rod was attached to an Anaheim Automation LS100 Series slide motor (Anaheim, CA), which had a 15" vertical range. Computer interfacing was outside the scope of this project, so slide motor height adjustments occurred manually. Connection between the dispensing needle and a syringe containing the polymer solution was achieved using Cole-Parmer 1/16" diameter TYGON lab tubing (Vernon Hills, IL). The tubing was attached to both the needle and the syringe using Cole-Parmer male and female 1/16" hose barbs. Solution flow rate was controlled with a Braintree Scientific (Braintree, MA) BS-8000 Series syringe pump capable of 0-99 ml/ hr volumetric rates.

A safety box was constructed in order to isolate the experimental system from the slide motor, syringe pump, and high voltage power supply. The box was 24"x24"x18" in size and was composed of 0.707" thick clear cast acrylic sheets manufactured by McMaster-Carr. A burgundy electrical grade 1/5" fiberglass sheet from McMaster-Carr was used as a contrast backing material for the safety box. As an extra precaution, McMaster-Carr adhesive backed polyester (PET) films were installed within the safety box to dissipate any build-up in electrostatic charges.

Electrospinning Solution Parameters. Polycaprolactone (m.w. = 80,000) from Sigma-Aldrich (St. Louis, MO) was used. PCL was prepared in 3-13% (w/v) solution concentrations. The solvents used in the solution parameters testing included acetone, toluene, chloroform, and dichloromethane. Once the electrospinning solution components were added together in a test tube, one to three hour sonications coupled with 60-80 °C hot water baths were used to thoroughly mix the electrospinning solutions to uniformity. Finally, the prepared solution was cooled to ambient temperature in order to yield a more consistent viscosity and surface tension during the electrospinning process.

Electrospinning Apparatus Parameters. Parameters such as voltage, needle to ground plate separation, syringe pump flow rate, needle gauge, and even ambient conditions were studied. The voltage output for the parameter optimization experiments varied between 5 kV and 30 kV. Similarly, experimental syringe pump flow rates ranged from approximately 0.10 ml/ hr to 15 ml/ hr, and the polymer solution dispensing needles varied from 18, 20, and 22 gauge sizes. Needle to ground plate separation distances ranged from 5 cm all the way to 20 cm.

Detection Methods. The series of PCL electrospinning parameter experiments employed the use of a Thermo Scientific compound light microscope and a Scanning Electron Microscope (SEM) to assess the physical morphology of the deposited electrospun fibers. Forty magnification optical zoom digital images from the compound light microscope were taken in order to qualitatively examine the deposited PCL fibers. The qualitative analysis from the digital images provided quick real-time feedback regarding the general morphology of the electrospun fibers. Decisions for experimental adjustments were based on these microscope images. The SEM was used as a secondary tool to provide a more detailed analysis of PCL fiber morphologies. Since SEM analysis could not be provided in real-time, only a few selected samples were examined using this method.

Solvent Effects. Acetone, toluene, chloroform, and dichloromethane were all tried as potential solvent for a PCL electrospinning solution. While each of the solvents had the ability to dissolve PCL pellets into solution, their performances in the electrospinning application varied greatly.

A 50% (v/v) electrospinning solution solvent composition of acetone/chloroform was used. The 50% (v/v) acetone/chloroform PCL solution prevented dispensing needle clogging, while evaporating fast enough to enable the formation of relatively uniform PCL fibers when electrospinning. Therefore, all subsequent electrospinning parameter experiments used the 50% (v/v) acetone to chloroform solvent.

Concentration and Needle Gauge Effects. Using the 50% (v/v) acetone to chloroform solvent composition for the PCL electrospinning solution, the effects of PCL concentration were tested. PCL concentrations ranged from 3-13% (w/v) during experimentation. Syringe pump flow rates ranged from 0.10 ml/ hr to 15 ml/ hr during electrospinning apparatus experimentation. All syringe pump flow rate experiments were conducted using the 8% (w/v) PCL in 50% (v/v) acetone to chloroform electrospinning solution. A 3 ml/hr volumetric flow rate was selected. Experimental voltage effect analysis was performed for the PCL electrospinning solution using an output voltage range between 5 kV and 30 kV. The voltage effect experiments used the 8% (w/v) PCL in 50% (v/v) acetone to chloroform electrospinning solution at a 3 ml/ hr flow rate. A 21 kV potential was selected. The final set of parameter adjustment experiments tested the effects of vertical plate separation between 5 cm and 20 cm. A separation distance of 15 cm between the needle tip and copper collection plate was selected.

Electrospinning Solution Aminolysis. The initial 2-step electrospinning solution aminolysis protocol is based on a protocol published by Gabriel et al. (J. Biomater. Sci. Polymer Edn. 2006, 17(5), 567-577), which provides a protocol to perform aminolysis on PCL films. According to the paper, optimal aminolysis is performed by soaking PCL films in an aminolysing solution consisting of 40% (v/v) ethylene diamine in distilled water. The films are incubated in the solution overnight at room temperature, and then washed in DI water for 2 hours. The protocol by Gabriel et al. was modified in order to perform aminolysis on PCL before the electrospinning process with the aim of electrospinning PCL fibers with amine functional groups already attached. The 2-step electrospinning solution aminolysis required two separate processes to form the final electrospinning solution. Essentially, an initial aminolysing solution consisting of 5% (w/v) PCL dissolved in 40% (v/v) ethylene diamine (EDA)/acetone was prepared and sonicated for two hours in a 60 °C hot water bath. At this point, the solution was either allowed to incubate at room temperature for up to 24 more hours or was immediately centrifuged and decanted to obtain the aminolysed PCL precipitate. If incubation was chosen, the solution was centrifuged and decanted after the prescribed incubation time. The aminolysed PCL precipitate was next washed with distilled, deionized $H_2O$ ($ddH_2O$) on an orbital shaker for two hours before being air-dried and weighed. The weighed aminolysed PCL precipitate was then re-dissolved in acetone to reconstitute a 5% (w/v) electrospinning solution. Later experimental runs used a chloroform solution to redissolve the aminolysed PCL precipitate for the electrospinning solution. The aminolysed PCL solutions were compared against themselves as well as a control PCL solution consisting of 5% wt/v PCL dissolved in acetone. The control solution underwent the same two hour sonication in an 80 °C hot water bath before electrospinning.

The baseline electrospinning parameters included an 18 gauge stainless steel blunt-end dispensing needle, a copper ground plate, 20 centimeter vertical needle to ground plate electrode separation, 21 kV potential, 1x3 inch glass slide for sample collection, and ~1 mL total solution deposition per sample. The solution flow rate was manually controlled by hand, and the electrode spacing and voltages were often altered during the experiment in order to obtain a fiber deposition for FT-IR analysis.

1-step Electrospinning Solution Aminolysis. The 1-step electrospinning solution aminolysis was developed with the goal of producing a PCL aminolysis solution that could be directly electrospun. This new method modified the 2-step aminolysis protocol by replacing acetone with chloroform in the aminolysing solution in order to eliminate the formation of a solid aminolysed PCL precipitate. The 1-step aminolysing solution consisted of 10% (w/v) PCL dissolved in 20-40% (v/v) EDA/chloroform. The 10% (w/v) solution replaced the 5% (w/v) solution because it was found that aminolysis significantly reduces solution viscosity, therefore necessitating a more viscous initial solution concentration in order to form electrospun fibers. The control solution consisted of 5% (w/v) PCL dissolved in 50 % (v/v) acetone/chloroform. All electrospinning solutions underwent two hour sonications at 80 °C before being electrospun. The 1-step aminolysis method did not include incubation after initial sonication. It was determined during the initial 2-step aminolysis tests that extensive loss of solution viscosity accompanies extended incubations of the aminolysing solutions.

Decellularized porcine diaphragm tendon was utilized in a test study application of the 1-step electrospinning solution aminolysis protocol. A detailed protocol for this study is provided in the Appendix. The study used the 1-step aminolysis electrospinning solution to directly electrospin onto five decellularized porcine diaphragm samples. The goal of the study was to determine if amine-functionalized PCL fibrous films would chemically or physically attach to the decellularized tissue scaffold. FT-IR solution and mesh analysis along with qualitative observation were utilized in the assessment of this study.

The baseline electrospinning parameters outlined in the 2-step aminolysis protocol were utilized in 1-step electrospinning solution aminolysis as well.

Electrospun Mesh Aminolysis. The electrospun mesh aminolysis protocols were carried out as a retrograde comparison to the 1-step and 2-step electrospinning solution aminolysis protocols. The electrospun meshes were produced from a control solution consisting of 5% (w/v) PCL in 50% (v/v) acetone to chloroform. The electrospinning parameters used in both the 2-step and 1-step aminolysis experiments were implemented here and were unchanged as well. However, unlike the 2-step and 1-step aminolysis solution experiments, the electrospinning parameters were kept constant during the course of the test runs. Each deposited electrospun mesh was produced from ~1 mL of electrospinning solution.

Electrospun PCL meshes were placed in variable aminolysing solution consisting of 0-100% (v/v) EDA in distilled water. The meshes were then incubated at room temperature for 24 hours, and then washed with distilled water on an orbital shaker for two hours before further analysis.

In another protocol, the PCL meshes were incubated for one hour in solutions of either 10wt.% EDA or 10wt.% hexamethylenediamine (HDA) in isopropyl alcohol. After incubation, the treated meshes were washed with distilled water for 2 hours on the orbital shaker before further analysis.

Detection Methods. The analysis conducted for the PCL aminolysis primarily focused on the use of FT-IR to determine the presence of amine functional group peaks. Figure 1 depicts the two discernable peak ranges for both the 1-step and 2-step aminolysis methods that differed from the control samples. One peak occurred in the 3201-3423 cm-1 range (peak 1). The other peak occurred in the 1508-1660 cm-1 range (peak 2). The degree of amine group functionalization was quantitatively measured as the area under each peak. A FT-IR scan of a washed drop-cast sample of the electrospinning solution was taken initially. Then, a final FT-IR scan was taken of the washed electrospun sample. All FT-IR samples were washed in ddH2O for two hours and allowed to air dry prior to a FT-IR scan. The mesh aminolysis samples were similarly analyzed with FT-IR, only without initial electrospinning solution scans.

FT-IR studies suggested that the degree of amine-group functionalization from the pre-electrospun solutions was not lost upon formation of the electrospun mesh products.

The details of the invention are also included in the attached power-point presentation titled "Acellular Porcine Tissue Crosslinked with Functionalized Nanomaterials: Novel Bionanocomposite Scaffolds for Soft Tissue Repair Applications," and the attached poster titled "A Novel, Crosslinked Bionanocomposite Material for Soft Tissue Repair Applications." Both attachments are hereby incorporated in their entireties.

While the invention has been described in connection with specific embodiments thereof, it will be understood that the inventive methodology is capable of further modifications. This patent application is intended to cover any variations, uses, or adaptations of the invention following, in general, the principles of the invention and including such departures from the present disclosure as come within known or customary practice within the art to which the invention pertains and as may be applied to the essential features herein before set forth and as follows in scope of the appended claims.

When introducing elements of the present invention or the embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above compositions and methods without departing from the scope of the invention, it is intended that all matter contained in the above description shall be interpreted as illustrative and not in a limiting sense.

## Claims

1. A bionanocomposite comprising:

   decellularized tissue; and nanomaterial crosslinked with the decellularized tissue and functionalized with surface functional groups capable of bonding with tissue;
   the nanomaterial is gold nanoparticles, gold nanorods, gold nanofibers, silver nanoparticles, silver nanorods, silver nanofibers, platinum nanoparticles, platinum nanorods, platinum nanofibers, titania nanoparticles, titania nanorods, titania nanofibers, silica nanoparticles, silica nanorods, silica nanofibers, alumina nanoparticles, alumina nanorods, alumina nanofibers, calcium phosphate nanoparticles, calcium phosphate nanorods, calcium phosphate nanofibers, BaTiO3 nanoparticles, BaTiO3 nanorods, BaTiO3 nanofibers, polycaprolactone nanofibers, polyglycolic acid nanofibers, polylactic acid nanofibers, polylacticglycolic acid nanofibers, polydoxanone nanofibers, trimethylene carbonate nanofibers, or combinations thereof;
   wherein the gold nanoparticles are functionalized with -COOH groups, -OH groups, methionine, mercaptomethylamine, mercaptoethylamine (MEA), mercaptopropylamine, mercaptobutylamine, or a combination thereof, the silicon carbide nanowires are functionalized with COOH groups, OH groups, aminopropyl-triethoxysilane, plasma polymerization with allyl amine, plasma polymerization with acrylic acid, plasma polymerization with hydroxyethyl methacrylate, and the polycaprolactone nanofibers are functionalized other than by aminolysis.

2. The bionanocomposite of Claim 1 wherein the gold nanoparticle is functionalized with -COOH groups, -OH groups, mercaptoethylamine, or a combination thereof.

3. The bionanocomposite of claim 1 or 2, wherein the bionanocomposite has a viscoelasticity as measured by the Young's modulus of from about 100 MPa to about 200 MPa.

4. The bionanocomposite of claim 3, wherein the bionanocomposite has a viscoelasticity as measured by the Young's modulus of from about 150 MPa to about 200 MPa.

5. The bionanocomposite of any one of claims 1 to 4 wherein the nanoparticles have a mean diameter from 5 nm to 50 nm.

6. The bionanocomposite of claim 5 wherein the nanoparticles have a mean diameter from 15 nm to 30 nm.

## Patentansprüche

1. Bionanoverbundstoff, der Folgendes umfasst:

   dezellularisiertes Gewebe; und
   Nanomaterial, das mit dem dezellularisierten Gewebe vernetzt und mit oberflächenfunktionellen Gruppen funktionalisiert ist, die sich mit Gewebe binden können;
   das Nanomaterial ist Goldnanopartikel, Goldnanostäbchen, Goldnanofasern, Silbernanopartikel, Silbernanostäbchen, Silbernanofasern, Platinnanopartikel, Platinnanostäbchen, Platinnanofasern, Titanoxidnanopartikel, Titanoxidnanostäbchen, Titanoxidnanofasern, Siliciumdioxidnanopartikel, Siliciumdioxidnanostäbchen, Siliciumdioxidnanofasern, Aluminiumoxidnanopartikel, Aluminiumoxidnanostäbchen, Aluminiumoxidnanofasern, Calciumphosphatnanopartikel, Calciumphosphatnanostäbchen, Calciumphosphatnanofasern, BaTiO3-Nanopartikel, BaTiO3-Nanostäbchen, BaTiO3-Nanofasern, Polycaprolactonnanofasern, Polyglykolsäurenanofasern, Polymilchsäurenanofasern, Polymilchglykolsäurenanofasern, Polydoxanonnanofasern, Trimethylencarbonatnanofasern oder Kombinationen davon;

wobei die Goldnanopartikel funktionalisiert sind mit -COOH Gruppen, -OH Gruppen, Methionin, Mercaptomethylamin, Mercaptoethylamin (MEA), Mercaptopropylamin, Mercaptobutylamin oder einer Kombination davon, die Siliciumcarbidnanodrähte funktionalisiert sind mit COOH-Gruppen, OH-Gruppen, Aminopropyltriethoxysilan, Plasmapolymerisation mit Allylamin, Plasmapolymerisation mit Acrylsäure, Plasmapolymerisation mit Hydroxyethylmethacrylat, und die Polycaprolactonnanofasern anders als durch Aminolyse funktionalisiert sind.

**2.** Bionanoverbundstoff nach Anspruch 1, wobei der Goldnanopartikel mit -COOH Gruppen, -OH Gruppen, Mercaptoethylamin oder einer Kombination davon funktionalisiert ist.

**3.** Bionanoverbundstoff nach Anspruch 1 oder 2, wobei der Bionanoverbundstoff eine Viskoelastizität, gemessen anhand des E-Moduls, von etwa 100 MPa bis etwa 200 MPa hat.

**4.** Bionanoverbundstoff nach Anspruch 3, wobei der Bionanoverbundstoff eine Viskoelastizität, gemessen anhand des E-Moduls, von etwa 150 MPa bis etwa 200 MPa hat.

**5.** Bionanoverbundstoff nach einem der Ansprüche 1 bis 5, wobei die Nanopartikel einen mittleren Durchmesser von 5 nm bis 50 nm haben.

**6.** Bionanoverbundstoff nach Anspruch 5, wobei die Nanopartikel einen mittleren Durchmesser von 15 nm bis 30 nm haben.

## Revendications

**1.** Bionanocomposite comprenant :

du tissu décellularisé ; et

du nanomatériau réticulé avec le tissu décellularisé et fonctionnalisé avec des groupes fonctionnels de surface capables de se lier avec du tissu ;

le nanomatériau est des nanoparticules d'or, des nanotiges d'or, des nanofibres d'or, des nanoparticules d'argent, des nanotiges d'argent, des nanofibres d'argent, des nanoparticules de platine, des nanotiges de platine, des nanofibres de platine, des nanoparticules de titanes, des nanotiges de titanes, des nanofibres de titanes, des nanoparticules de silice, des nanotiges de silice, des nanofibres de silice, des nanoparticules d'alumine, des nanotiges d'alumine, des nanofibres d'alumine, des nanoparticules de phosphate de calcium, des nanotiges de phosphate de calcium, des nanofibres de phosphate de calcium, des nanoparticules de BaTiO3, des nanotiges de BaTiO3, des nanofibres de BaTiO3, des nanofibres de polycaprolactone, des nanofibres d'acide polyglycolique, des nanofibres d'acide polylactique, des nanofibres d'acide polylactique-glycolique, des nanofibres de polydoxanone, des nanofibres de carbonate de triméthylène, ou des combinaisons de celles-ci ;

dans lequel les nanoparticules d'or sont fonctionnalisées avec des groupes -COOH, des groupes -OH, de la méthionine, de la mercaptométhylamine, de la mercaptoéthylamine (MEA), de la mercaptopropylamine, de la mercaptobutylamine, ou une combinaison de ceux-ci, les nanofils de carbure de silicium sont fonctionnalisés avec des groupes COOH, des groupes OH, de l'aminopropyle-triéthoxysilane, la polymérisation au plasma avec de l'amine allylique, la polymérisation au plasma avec de l'acide acrylique, la polymérisation au plasma avec du méthacrylate d'hydroxyéthyle, et les nanofibres de polycaprolactone sont fonctionnalisées autrement que par aminolyse.

**2.** Bionanocomposite selon la revendication 1 dans lequel la nanoparticule d'or est fonctionnalisée avec des groupes -COOH, des groupes -OH, de la mercaptoéthylamine, ou une combinaison de ceux-ci.

**3.** Bionanocomposite selon selon la revendication 1 ou 2, dans lequel, dans lequel le bionanocomposite a une viscoélasticité telle que mesurée par le module de Young d'environ 100 MPa à environ 200 MPa.

**4.** Bionanocomposite selon la revendication 3, dans lequel le bionanocomposite a une viscoélasticité telle que mesurée par le module de Young d'environ 150 MPa à environ 200 MPa.

**5.** Bionanocomposite selon l'une quelconque des revendications 1 à 4, dans lequel les nanoparticules ont un diamètre moyen de 5 nm à 50 nm.

6. Bionanocomposite selon la revendication 5, dans lequel les nanoparticules ont un diamètre moyen de 15 nm à 30 nm.

# FIG. 1A

4X 5.0kV 4.9mm x50.0k SE(U) 1/3/07                    1.00um

# FIG. 1B

1X 5.0kV 13.1mm x50.0k SE(U) 1/18/07                    1.00um

## FIG. 2A

Decellurized Tissue

DiaSiCNW 5.0kV 7.9mm x5.00k SE(U)          10.0um

Crosslinked Tissue (SiCNW on Surface)

## FIG. 2B

DiaSiCNW 5.0kV 7.9mm x15.0k SE(U)          3.00um

Crosslinked Tissue (SiCNW Close-Up)

# FIG. 3A

Crosslinked Tissue (AuNP Cluster Inside)

# FIG. 3B

Crosslinked Tissue (AuNP Cluster Close-Up)

# FIG. 4

# FIG. 5

# FIG. 6

DNA content over time

# FIG. 7

GAG normalized for DNA content over time

## FIG. 8A

## FIG. 8B

FIG. 9

FIG. 10A

# FIG. 10B

# FIG. 10C

## FIG. 10D

## FIG. 10E

## FIG. 11A

## FIG. 11B

# FIG. 11C

# FIG. 11D

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006257377 A

### Non-patent literature cited in the description

- **GILBERT et al.** Decellularization of tissues and organs. *Biomaterials,* 2006, vol. 27, 3675-3683
- **WEBSTER TJ et al.** *J Biomed Mater Res,* 2000, vol. 51, 475-483
- **PRICE RL et al.** *Journal of Biomedical Materials Research,* 2003, vol. 67A, 1284-1293
- **WEBSTER TJ et al.** *Biomaterials,* 2004, vol. 25, 4731-4739
- **PARK GE et al.** *Biomaterials,* 2005, vol. 26, 3075-3082
- **THAPA A et al.** *Journal of Biomedical Materials Research,* 2003, vol. 67A, 1374-1383
- **CHRISTENSON EM et al.** *Journal of Orthopaedic Research,* 2007, vol. 25, 11-22
- **S.K. WILLIAMS et al.** Covalent modification of porous implants using extracellular matrix proteins to accelerate neovascularization. *J Biomed Mater Res,* 2006, vol. 78A, 59-65
- **HSU et al.** *J. Biomedical Materials Research,* 2006, vol. 759
- **BELLINO MG et al.** *Physical Chemistry Chemical Physics,* 2004, vol. 6, 424-428
- **DUAN X et al.** *Journal of Biomedical Materials Research,* 2005, vol. 75, 510-518
- **COOK JL et al.** *American Journal of Veterinary Research,* 2008, vol. 69, 148-156
- **CHRISTENSON EM et al.** Nanobiomaterial applications in orthopedics. *Journal of Orthopaedic Research,* 2007, vol. 25, 11-22
- **BALASUNDARAM G ; WEBSTER TJ.** A perspective on nanophase materials for orthopedic implant applications. *Journal of Materials Chemistry,* 2006, vol. 16, 3737-3745
- **DILLOW AK ; LOWMAN AM.** Biomimetic Materials and Design. Marcel Dekker, Inc, 2002, 29-53
- **KAY S ; THAPA A ; HABERSTROH KM ; WEBSTER TJ.** Nanostructured polymer/nanophase ceramic composites enhance osteoblast and chondrocyte adhesion. *Tissue Engineering,* 2002, vol. 8, 753-761
- **WEBSTER TJ ; ERGUN C ; DOREMUS RH ; SIEGEL RW ; BIZIOS R.** Specific proteins mediate enhanced osteoblast adhesion on nanophase ceramics. *Journal of Biomedical Materials Research,* 2000, vol. 51, 475-483
- **WEBSTER TJ ; SCHADLER LS ; SIEGEL RW ; BIZIOS R.** Mechanisms of enhanced osteoblast adhesion on nanophase alumina involve vitronectin. *Tissue Engineering,* 2001, vol. 7, 291-301
- **ABRAHAM GA ; MURRAY J ; BILLIAR K ; SULLIVAN SJ.** Evaluation of the porcine intestinal collagen layer as a biomaterial. *Journal of Biomedical Materials Research,* 2000, vol. 51, 442-452
- **CHANG Y ; TSAI CC ; LIANG HC ; SUNG HW.** In vivo evaluation of cellular and acellular bovine pericardia fixed with a naturally occurring crosslinking agent (genipin). *Biomaterials,* 2002, vol. 23, 2447-245
- **KHOR E.** Methods for the treatment of collagenous tissues for bioprostheses. *Biomaterials,* 1997, vol. 18, 95-105
- **HSU SH ; TANG CM ; TSENG HJ.** Biocompatibility of poly(ether)urethane-gold nanocomposites. *Journal of Biomedical Materials Research,* 2006, vol. 79, 759-770
- **VALENTIN JE ; BADYLAK JS ; MCCABE GP ; BADYLAK SF.** Extracellular matrix bioscaffolds for orthopaedic applications. A comparative histologic study. *Journal of Bone and Joint Surgery American,* 2006, vol. 88, 2673-2686
- **GABRIEL et al.** *J. Biomater. Sci. Polymer Edn.,* 2006, vol. 17 (5), 567-577